(19) Europäisches Patentamt
European Patent Office
Office européen des brevets

(11) EP 4 534 087 A1

(12) EUROPEAN PATENT APPLICATION
published in accordance with Art. 153(4) EPC

(43) Date of publication:
**09.04.2025 Bulletin 2025/15**

(21) Application number: **23815191.4**

(22) Date of filing: **30.05.2023**

(51) International Patent Classification (IPC):
*A61K 31/4985* (2006.01) *A61K 31/496* (2006.01)
*A61K 31/551* (2006.01) *A61K 31/517* (2006.01)
*A61K 31/519* (2006.01) *A61K 31/47* (2006.01)
*A61K 31/44* (2006.01) *A61P 35/00* (2006.01)

(52) Cooperative Patent Classification (CPC):
**A61K 31/44; A61K 31/47; A61K 31/496; A61K 31/4985; A61K 31/517; A61K 31/519; A61K 31/551; A61P 35/00**

(86) International application number:
**PCT/CN2023/097041**

(87) International publication number:
**WO 2023/232012 (07.12.2023 Gazette 2023/49)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.05.2022 CN 202210597667**

(71) Applicants:
- **CSPC Zhongqi Pharmaceutical Technology (Shijiazhuang) Co., Ltd.
Shijiazhuang, Hebei 050035 (CN)**
- **Hangzhou Innogate Pharma Co., Ltd.
Hangzhou, Zhejiang 311121 (CN)**
- **CSPC OUYI Pharmaceutical Co.Ltd
Shijiazhuang, Hebei 052165 (CN)**

(72) Inventors:
- **YANG, Hanyu
Shijiazhuang, Hebei 050035 (CN)**
- **DAN, Mo
Shijiazhuang, Hebei 050035 (CN)**
- **LI, Yanling
Shijiazhuang, Hebei 050035 (CN)**
- **ZHANG, Hancheng
Hangzhou, Zhejiang 311121 (CN)**
- **LIU, Xibao
Shijiazhuang, Hebei 050035 (CN)**
- **CAI, Congcong
Hangzhou, Zhejiang 311121 (CN)**
- **LIU, Jieru
Shijiazhuang, Hebei 050035 (CN)**
- **WU, Xiaojuan
Shijiazhuang, Hebei 050035 (CN)**

(74) Representative: **Mewburn Ellis LLP
Aurora Building
Counterslip
Bristol BS1 6BX (GB)**

(54) **PHARMACEUTICAL COMBINATION AND PHARMACEUTICAL COMPOSITION FOR TREATING CANCER**

(57) Provided are a pharmaceutical combination and a pharmaceutical composition comprising a compound of formula (I) or a pharmaceutically acceptable form thereof and at least one other anticancer agent, and use thereof in the preparation of a medicine for treating a malignant tumor disease. The other anticancer agent is a CDK inhibitor or a pharmaceutically acceptable salt thereof (such as palbociclib), lenvatinib or a pharmaceutically acceptable salt thereof, sorafenib or a pharmaceutically acceptable salt thereof, or any combination of the above.

EP 4 534 087 A1

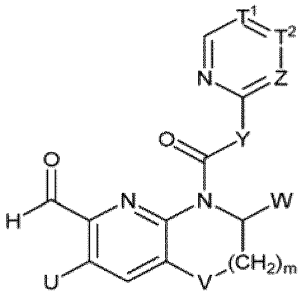

(I)

## Description

### CROSS-REFERENCE TO RELATED DISCLOSURE

The disclosure claims the priorities to and benefits of the Chinese Patent Application No. 202210597667.9 filed with the China National Intellectual Property Administration on May 30, 2022, which is incorporated herein by reference in its entirety.

### TECHNICAL FIELD

The disclosure belongs to the field of medicine, and specifically relates to the use of a FGFR4 inhibitor represented by formula (I) combined with at least one other anticancer agent for the treatment of malignant tumor diseases.

### BACKGROUND

Fibroblast growth factor receptor 4 (FGFR4) is a protein encoded by the FGFR4 gene in a human body. The protein is one of the members of the fibroblast growth factor receptor family, and the amino acid sequences among the members of the family are highly conserved throughout evolution. FGFR family members 1 to 4 differ from each other in their ligand affinity and tissue distribution. A representative full-length protein consists of: an ectodomain composed of three immunoglobulin-like domains, a single hydrophobic membrane-spanning segment, and a cytoplasmic tyrosine kinase domain. An extracellular portion of the protein interacts with a fibroblast growth factor, initiating a cascade of downstream signals, ultimately influencing mitogenesis and differentiation. A genomic structure of the FGFR4 gene contains eighteen exons. Although alternative splicing has been observed, there is no evidence to confirm that the C-terminal half of the IgIII domain of this protein varies among three alternative forms, as shown in FGFR1-3.

To date, there is no approved potent and selective FGFR4 inhibitor. While several FGFR inhibitors are currently being used in clinical trials for the treatment of cancers with FGFR1-3 aberrations, many of these inhibitors exhibit promiscuous kinase activity or moderate to weak efficacy against FGFR4. Lack of kinome selectivity may result in toxicity caused by the off-target effect. Specifically, on-target dose-limiting toxicity has been observed in both animals and patients administered with FGFR1 and FGFR3 inhibitors. For example, ectopic mineralization, characterized by inappropriate calcium-phosphorus deposition in soft tissue, has been observed in rats treated with a FGFR1 inhibitor. Inhibition of FGFR1 and FGFR3 may also lead to hyperphosphatemia. This suggests that selective inhibition of FGFR4 without inhibition of other isoforms of FGFR, including FGFR1 and FGFR3, may be desirable in order to avoid certain toxicities. FGFR4 preferentially binds to fibroblast growth factor 19 (FGF19). For example, aberrant signaling via fibroblast growth factor 19 (FGF19)-FGFR4 signaling complex has been verified to result in hepatocellular carcinoma (HCC) in mice and has already been implicated to play a similar role in humans.

A malignant tumor (cancer) is a major disease that endangers people's life and health. In recent years, with rapid development of tumor biology and related disciplines, specific antineoplastic drugs targeting abnormal signal system targets in tumor cells are the focus of new drug development. At the same time, the combination of various antineoplastic drugs for the treatment of tumor diseases is also a hot topic of scientific research.

Liver cancer is one of the malignant tumors with the highest incidence rate and mortality, with 466 thousand new cases of liver cancer and 422 thousand deaths per year in China. Studies have shown that the FGF19-FGFR4 signaling system is closely associated with hepatocellular carcinoma (HCC), wherein FGFR4 is a FGFR subtype that is highly expressed in human hepatocytes and multiple variants of FGFR4 are found in hepatocellular carcinoma patients. In addition, the ligand FGF19 also has mutations such as amplification in many hepatocellular carcinomas, resulting in abnormal activation of the FGFR4 signaling pathway. Selective inhibition of FGFR4 without inhibition of other subtypes FGFR1, FGFR2, and FGFR3 avoids certain toxicities and is likely to be an important target for the treatment of liver cancer. Clinical studies have shown that FGFR inhibitors can be used in the treatment of a variety of cancers, but there is an urgent need to develop a selective FGFR4 inhibitor for the treatment of a variety of tumors, particularly the treatment of liver cancer.

Chinese patent application CN108948004A discloses a FGFR4 inhibitor as represented by the following formula (I):

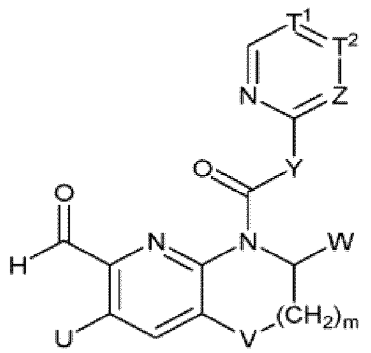

(I) .

Palbociclib, with the chemical name of (6-acetyl-8-cyclopentyl-5-methyl-2-([5-(piperazin-1-yl)pyridin-2-yl]amino}pyrido[2,3-d]pyrimidin-7(8H)-one), is an inhibitor of cyclin-dependent kinases (CDK) 4 and CDK6 approved by FDA. Palbociclib has the following structure:

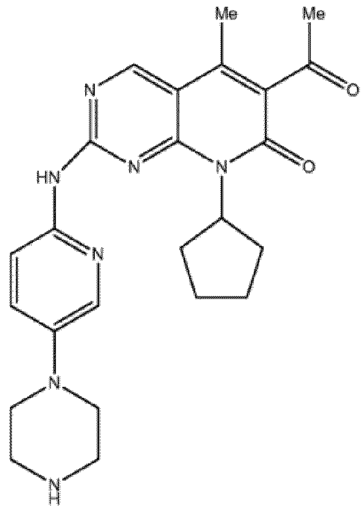

.

Palbociclib, with the trade name of IBRANCE®, has marketing approval indications including that: the product is used for locally advanced or metastatic breast cancer with hormone receptor (HR) positive and human epidermal growth factor receptor 2 (HER2) negative. It should be used in combination with an aromatase inhibitor as initial endocrine therapy in a postmenopausal female patient. The recommended dose of palbociclib is 125 mg, once daily for 21 consecutive days, followed by discontinuation for 7 days (3/1 administration regimen) in a treatment cycle of 28 days. Treatment should be continued unless the patient no longer achieves clinical benefit or develops unacceptable toxicity. Temporarily interrupted/delayed administration and/or reduced dose, or permanently discontinued administration may be required when occurring certain adverse reactions. The first reduced dose is 100 mg/day. The second reduced dose is 75 mg/day. If a further reduction of dose to less than 75 mg/day is required, the treatment should be terminated. In clinical studies, the most common (20%) adverse reactions at any grade reported in a patient treated with palbociclib are neutropenia, infection, leukopenia, fatigue, nausea, stomatitis, anemia, alopecia, and diarrhea. The most common (2%) adverse reactions (≥ grade 3) of palbociclib are neutropenia, leukopenia, anemia, fatigue and infection. In safety studies of treatment with palbociclib (125 mg/day) in combination with letrozole (2.5 mg/day) versus treatment with placebo in combination with letrozole, 36% of patients treated with palbociclib in combination with letrozole are subject to reduction of dose due to adverse reactions at any grade. 43/444 (9.7%) of patients treated with palbociclib in combination with letrozole and 13/222 (5.9%) of patients treated with placebo in combination with letrozole experience permanent drug withdrawal due to adverse reactions. Adverse reactions resulting in permanent drug withdrawal in patients treated with palbociclib in combination with letrozole include neutropenia (1.1%) and elevation of alanine aminotransferase (0.7%). (Source: Palbociclib Capsule (Trade Name: IBRANCE) Package Insert).

Lenvatinib, with chemical name of (4-[3-chloro-4-(N'-cyclopropylureido) phenoxy]-7-methoxyquinoline-6-carboxamide), is a multi-target receptor tyrosine kinase (RTK) inhibitor that inhibits the kinase activity of vascular endothelial growth factor (VEGF) receptors VEGFR1 (FLT1), VEGFR2 (KDR), and VEGFR3 (FLT4). Lenvatinib also inhibits other RTKs, which are associated with pathogenicity angiogenesis, tumor growth, and cancer progression, in addition to normal

cellular functions, including the fibroblast growth factor (FGF) receptors FGFR1, FGFR2, FGFR3, and FGFR4; platelet-derived growth factor receptor α (PDGFRα), KIT and RET. Lenvatinib has the following structure:

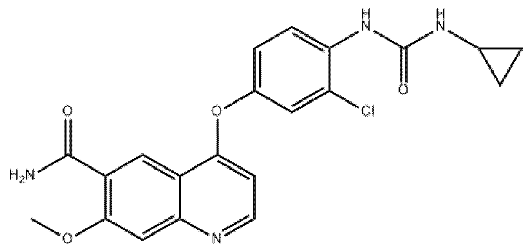

.

The mesylate salt of lenvatinib (i.e., lenvatinib mesylate), with chemical name of 4-[3-chloro-4-(N'-cyclopropylureido)phenoxy]-7-methoxyquinoline-6-carboxamide mesylate, has the following structure:

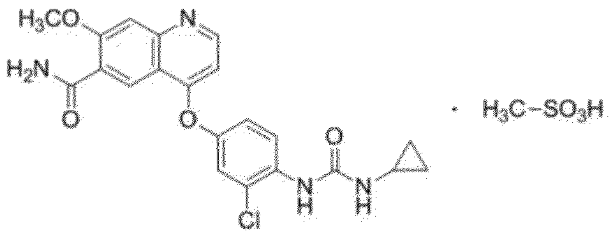

.

Lenvatinib mesylate, with the trade name of LENVIMA®, has a marketing approval indication that: the product is used for patients with unresectable hepatocellular carcinoma who have not been subjected to systemic therapy in the past (the pivotal study of this product excludes patients with hepatocellular carcinoma who are amenable to local therapy, for whom there are no available study data). For patients weighing <60 kg, the recommended daily dose of this product is 8 mg (2*4 mg capsules) once daily; for patients weighing ≥60 kg, the recommended daily dose is 12 mg (3*4 mg capsules) once daily. Treatment should be continued until disease progression or intolerable toxic side effects occur. In REFLECT study, most patients (99%) in the lenvatinib group experience at least one adverse reaction. The most common side effects and adverse reactions (≥20%) of lenvatinib are: hypertension (45%), fatigue (44%), diarrhea (39%), decreased appetite (34%), weight loss (31%), arthralgia/myalgia (31%), abdominal pain (30%), palmar-plantar erythema syndrome (27%), proteinuria (26%), bleeding events (25%), dysphonia (24%), hypothyroidism (21%), and nausea (20%). Grade 3 or higher adverse reactions occur in 75% of patients in the lenvatinib group. The most common grade 3 or higher side effects and adverse reactions in patients treated with lenvatinib (≥5%) are: hypertension (24%), weight loss (8%), fatigue (7%), elevated bilirubin (7%), proteinuria (6%), thrombocytopenia (5%), hepatic encephalopathy (5%), elevated gamma-glutamyl transferase (5%), bleeding events (5%), and elevated aspartate aminotransferase (5%).

The most common serious adverse reactions (≥2%) in patients treated with lenvatinib are: bleeding events (5%), hepatic encephalopathy (5%), liver failure (3%), ascites (3%), and loss of appetite (2%). Adverse reactions result in dose reduction or interruption in 62% of patients treated with lenvatinib. The most common adverse reactions (≥5%) resulting in dose reduction or interruption in the lenvatinib treatment group are: fatigue (10%), decreased appetite (8%), diarrhea (8%), proteinuria (7%), hypertension (6%), and palmar-plantar erythema syndrome (5%). The most common adverse reactions (≥1%) resulting in lenvatinib interruption are: fatigue (2%), bleeding events (2%), hepatic encephalopathy (2%), hyperbilirubinemia (1%), and liver failure (1%). (Source: Lenvatinib Mesylate Capsule (Trade Name: LENVIMA) Package Insert).

Sorafenib is a multi-target kinase inhibitor. In vitro tests have shown that it may inhibit tumor cell proliferation and anti-angiogenic effects. Sorafenib inhibits target sites CRAF, BRAF, BRAF V600E, c-Kit, FLT-3 of tumor cells and target sites CRAF, VEGFR-2, VEGFR-3, PDGFR-β of tumor vascular. RAF kinases are serine/threonine kinases, while c-Kit, FLT-3, VEGFR-2, VEGFR-3, PDGFR-β are tyrosine kinases, which act on tumor cell signaling pathways, angiogenesis, and apoptosis. In vitro tests have shown that sorafenib may inhibit tumor growth and angiogenesis in a variety of human cancer cell xenograft tumor nude mouse models, such as human hepatocellular carcinoma, renal cell carcinoma. Sorafenib has the following structure:

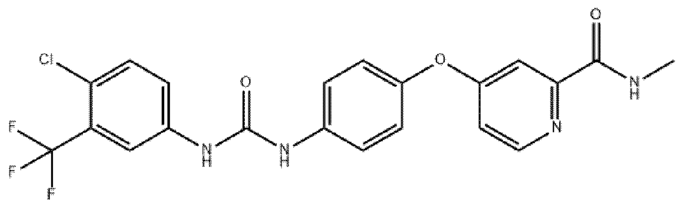

.

Sorafenib tosylate, with chemical name of 4-{4-[3-(4-chloro-3-trifluoromethyl-phenyl)-acylurea]-phenoxy}-pyridine-2-carboxylic acid methylamide-4-toluenesulfonate, has the following structure:

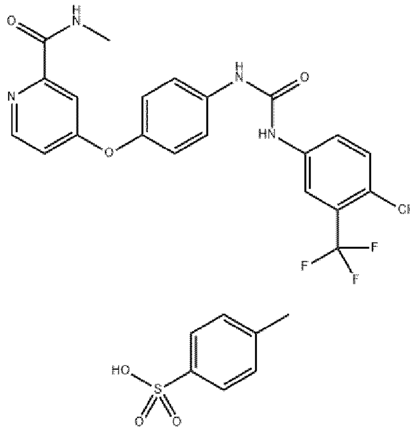

.

Indications approved for marketed sorafenib tosylate include inoperable or distally metastatic hepatocellular carcinoma (there is currently lack of randomized control clinical study data comparing sorafenib to interventional therapies such as transhepatic arterial chemotherapy and embolization (TACE) in patients with advanced hepatocellular carcinoma, so it is not clear whether this product is superior or inferior to interventional therapy, nor is it clear whether using sorafenib is beneficial for patients who have received interventional therapy in the past. Doctors are advised to choose specific treatment considering patient's particular case). The recommended dose of sorafenib is 0.4 g (2×0.2 g) per time, twice daily, and treatment should be continued until the patient fails to achieve clinical benefit or develops an intolerable toxic reaction. Treatments of suspected adverse reactions involve suspending or reducing the dose of sorafenib, if dose reduction is required, the dose of sorafenib is reduced to 0.4 g (2×0.2 g) per time, once daily. The most common drug-related adverse events reported in the safety evaluation of a Phase II clinical study that included 638 patients treated with sorafenib (including 202 patients with renal cell carcinoma, 137 patients with hepatocellular carcinoma and 299 patients with other cancers) are: rash (38%), diarrhea (37%), hand and foot skin reactions (35%), and malaise (33%). Among these, the incidence of drugs at CTC (version V2.0) Grade 3 and Grade 4-related adverse events is 37% and 3%, respectively. In Bayer-sponsored clinical study, the incidence of congestive heart failure in patients taking sorafenib is 1.9% (N=2276). In patients with lung cancer, mortality rate is higher when receiving a chemotherapy regimen of sorafenib in combination with dual platinum than when receiving a chemotherapy regimen of only dual platinum (carboplatin/paclitaxel and gemcitabine/cisplatin). The exact reason for this result remains unclear. (Source: Sorafenib Tosylate Tablets (Trade Name: Nexavar) Package Insert).

Chinese patent application CN110022900A (the specific compound is a compound of the following formula (1)) and Chinese patent application CN109803684A (the specific compound is a compound of the following formula (2)) both report a combination of a FGFR4 inhibitor with a CDK inhibitor, in particular a combination with palbociclib. Chinese patent application CN111787922 reports a combination of a FGFR4 inhibitor (the specific compound is a compound of the following formula (2)) and lenvatinib. In liver cancer experimental models, the FGFR inhibitors reported in the above patents are administered in large doses, ranging from as low as 30 mg/kg bid to as high as 500 mg/kg qd, posing a potential risk to the safety of the combination of drugs.

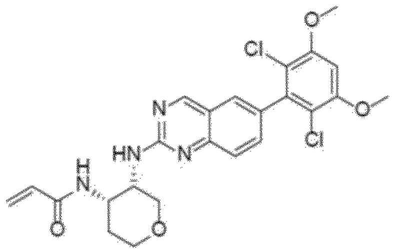 (1), 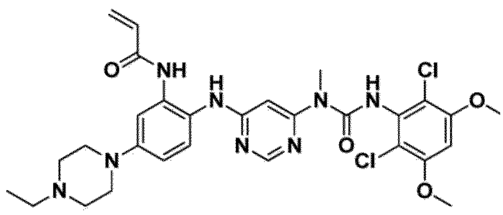 (2)

In view of the above, there is a need in the art for combination therapies with significant efficacy and high safety for the treatment of cancer, such as liver cancer.

## SUMMARY OF INVENTION

The inventors found that a FGFR4 inhibitor represented by formula (I), in particular a compound of formula (B) as shown below, is administered at a low dose in combination with a CDK inhibitor (such as palbociclib) or a pharmaceutically acceptable salt thereof, lenvatinib or a pharmaceutically acceptable salt thereof, sorafenib or a pharmaceutically acceptable salt thereof, or any combination of the foregoing, to treat cancer, such as liver cancer, which achieves a significant therapeutic effect compared to single-drug therapy and is safe to administer.

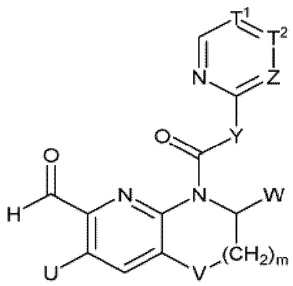 (I), 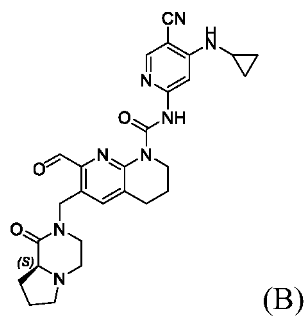 (B)

In the first aspect, the disclosure provides a pharmaceutical combination, comprising a compound of formula (I) or a pharmaceutically acceptable form thereof and at least one other anticancer agent, wherein the other anticancer agent is selected from a CDK inhibitor or a pharmaceutically acceptable salt thereof, lenvatinib or a pharmaceutically acceptable salt thereof, sorafenib or a pharmaceutically acceptable salt thereof, or any combination of the foregoing,

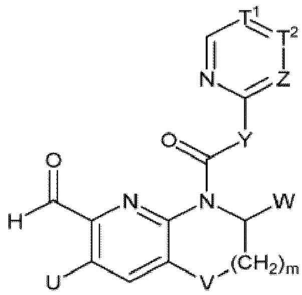 (I),

wherein:

$T^1$ is $CR^1$, wherein $R^1$ is cyano;
$T^2$ is $CR^2$, wherein $R^2$ is selected from the group consisting of:

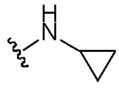 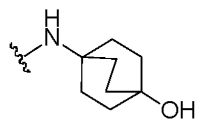 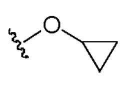 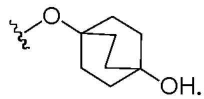,

Z is CH;
Y is NR, wherein R is hydrogen;
W is hydrogen;
V is $CH_2$;
U is selected from the group consisting of:

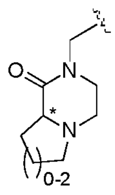 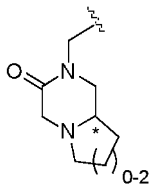 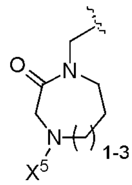 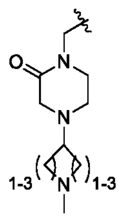 ;

wherein $X^5$ is $C_{1-4}$ alkyl;
m is 1.

In an embodiment, the pharmaceutical combination may comprise the compound of formula (I) or a pharmaceutically acceptable form thereof as a first dosage form, and the first dosage form of the compound of formula (I) or a pharmaceutically acceptable form thereof may further comprise at least one pharmaceutically acceptable carrier, vehicle, or excipient.

In an embodiment, the pharmaceutical combination may comprise the other anticancer agent as a second dosage form, and the second dosage form of the other anticancer agent may further comprise at least one pharmaceutically acceptable carrier, vehicle, or excipient.

In an embodiment, the first dosage form and the second dosage form may be the same as or different from each other.

In an embodiment, the first dosage form and the second dosage form may each be a single-dose dosage form or a divided-dose dosage form.

In an embodiment, the first dosage form of the compound of formula (I) or a pharmaceutically acceptable form thereof, and the second dosage form of the other anticancer agent may be administered simultaneously or separately. The administration may be oral administration, injection administration, topical administration or in vitro administration.

In an embodiment, the pharmaceutical combination can be used to treat a malignant tumor disease.

For example, in an embodiment, the compound of formula (I) or a pharmaceutically acceptable form thereof may improve the efficacy of the other anticancer agent in the treatment of a malignant tumor disease, e.g., the compound of formula (I) or a pharmaceutically acceptable form thereof may (at a low dose) enhance the efficacy of the other anticancer agent; or the compound of formula (I) or a pharmaceutically acceptable form thereof may reduce a side effect or the like of the other anticancer agent.

Thus, the disclosure also provides the use of the pharmaceutical combination in preparation of a medicine for treating a malignant tumor disease.

For example, provided is the use of a compound of Formula (I) or a pharmaceutically acceptable form thereof in combination with at least one other anticancer agent in preparation of a medicine for treating a malignant tumor disease, the other anticancer agent is a CDK inhibitor or a pharmaceutically acceptable salt thereof, lenvatinib or a pharmaceutically acceptable salt thereof, sorafenib or a pharmaceutically acceptable salt thereof, or any combination of the foregoing, wherein formula (I) is as defined above.

For example, provided is the use of a compound of formula (I) or a pharmaceutically acceptable form thereof in preparation of a medicine for treating a malignant tumor disease, wherein the medicine comprises at least one other anticancer agent, wherein the other anticancer agent is a CDK inhibitor or a pharmaceutically acceptable salt thereof, lenvatinib or a pharmaceutically acceptable salt thereof, sorafenib or a pharmaceutically acceptable salt thereof, or any combination of the foregoing, wherein formula (I) is as defined above. Specifically, provided is the use of a compound of formula (I) or a pharmaceutically acceptable form thereof in preparation of a medicine for improving the efficacy of other anticancer agent for treating a malignant tumor disease, wherein the other anticancer agent is a CDK inhibitor or a pharmaceutically acceptable salt thereof, lenvatinib or a pharmaceutically acceptable salt thereof, sorafenib or a pharmaceutically acceptable salt thereof, or any combination of the foregoing, wherein formula (I) is as defined above.

In the second aspect, the disclosure provides a pharmaceutical composition comprising a compound of formula (I) or a pharmaceutically acceptable form thereof, at least one other anticancer agent and at least one pharmaceutically acceptable carrier, vehicle, or excipient, wherein the other anticancer agent is a CDK inhibitor or a pharmaceutically acceptable salt thereof, lenvatinib or a pharmaceutically acceptable salt thereof, sorafenib or a pharmaceutically acceptable salt thereof, or any combination of the foregoing, wherein formula (I) is as defined above.

In an embodiment, the pharmaceutical composition comprises a therapeutically effective amount of the compound of formula (I) or a pharmaceutically acceptable form thereof.

In an embodiment, the pharmaceutical composition may be used to treat a malignant tumor disease.

Therefore, the disclosure also provides the use of the pharmaceutical composition in preparation of a medicine for treating a malignant tumor disease.

In the third aspect, the disclosure provides a method for treating a malignant tumor disease, comprising administering to a subject in need thereof a therapeutically effective amount of the above pharmaceutical combination or the above pharmaceutical composition.

In an embodiment, the disclosure provides a method for improving the efficacy of other anticancer agent for treating a malignant tumor disease, comprising administering to a subject in need thereof a therapeutically effective amount of a compound of Formula (I) or a pharmaceutically acceptable form thereof, wherein the other anticancer agent is a CDK inhibitor or a pharmaceutically acceptable salt thereof, lenvatinib or a pharmaceutically acceptable salt thereof, sorafenib or a pharmaceutically acceptable salt thereof, or any combination of the foregoing, wherein formula (I) is as defined above.

In an embodiment, the method further comprises determining whether the subject suffers from the malignant tumor disease, or receiving information that the subject suffers from the malignant tumor disease, before administering the pharmaceutical combination or the pharmaceutical composition, wherein the malignant tumor disease is characterized by at least one biomarker selected from: fibroblast growth factor 19 (FGF19) overexpression, FGF19 amplification and fibroblast growth factor receptor 4 (FGFR4) overexpression.

In an embodiment, the method further comprises identifying that the subject has response to the administration of the pharmaceutical combination or the pharmaceutical composition after determining whether the subject suffers from the malignant tumor disease or receiving information that the subject suffers from the malignant tumor disease.

Specifically, the disclosure provides a method for treating a malignant tumor disease in a patient in need thereof, comprising:

a) determining whether a patient suffers from a malignant tumor disease or receiving information that a patient suffers from a malignant tumor disease, wherein the malignant tumor disease is characterized by at least one biomarker selected from: fibroblast growth factor 19 (FGF19) overexpression, FGF19 amplification and fibroblast growth factor receptor 4 (FGFR4) overexpression;

b) identifying that the patient has response to the administration of the above pharmaceutical combination or the above pharmaceutical composition; and

c) administering the above pharmaceutical combination or the above pharmaceutical composition to the patient.

In the fourth aspect, the disclosure provides a kit comprising the above pharmaceutical combination or the above pharmaceutical composition, and an instruction for use.

In an embodiment, the kit may be used for any of the foregoing uses or methods.

In some embodiments, the kit comprises a therapeutically effective amount of a compound of Formula (I) or a pharmaceutically acceptable form thereof and at least one other anticancer agent, wherein the other anticancer agent is a CDK inhibitor or a pharmaceutically acceptable salt thereof, lenvatinib or a pharmaceutically acceptable salt thereof, sorafenib or a pharmaceutically acceptable salt thereof, or any combination of the foregoing, wherein formula (I) is as defined above.

The kit may comprise a therapeutically effective amount of a compound of formula (I) or a pharmaceutically acceptable form thereof and at least one other anticancer agent in a separated container (e.g., a vial, a ampoule, a bottle, a tank, a syringe and/or a dispenser package, a flexible package (e.g., seled Mylar or plastic package) or other suitable container), wherein the other anticancer agent is a CDK inhibitor or a pharmaceutically acceptable salt thereof, lenvatinib or a pharmaceutically acceptable salt thereof, sorafenib or a pharmaceutically acceptable salt thereof, or any combination of the foregoing, wherein the compound of formula (I) or a pharmaceutically acceptable form thereof and at least one other anticancer agent may each be formulated to an individual dosage form, or formulated to a compounded dosage form. In some embodiments, the compound of formula (I) or a pharmaceutically acceptable form thereof and at least one other anticancer agent may be present in separate containers or in a single container. In some embodiments, the provided kit may optionally further comprise one or more additional containers comprising one or more pharmaceutical excipients for diluting or suspending a therapeutically effective amount of a compound of formula (I) or a pharmaceutically acceptable form thereof and/or at least one other anticancer agent. In some embodiments, prior to administration, a therapeutically effective amount of a compound of Formula (I) or a pharmaceutically acceptable form thereof and at least one other anticancer agent each contained in a separate container are combined (optionally in a third container comprising a pharmaceutical excipient for diluting or suspending) to form a unit dosage form.

In some embodiments, the kit further includes an instruction for any use or method in accordance with the disclosure. The kit may further comprise an instruction for use that describes selecting individual suitable for treatment

(e.g., describes how to combine a compound of formula (I) or a pharmaceutically acceptable form thereof and at least one other anticancer agent into a single dose form (wherein the other anticancer agent is selected from a CDK inhibitor or a pharmaceutically acceptable salt thereof, lenvatinib or a pharmaceutically acceptable salt thereof, sorafenib or a pharmaceutically acceptable salt thereof, or any combination of the foregoing), the type of cancer for which the kit is indicated, the frequency of administration of a compound of formula (I) or a pharmaceutically acceptable form thereof as a separate dosage form, and other information relating to co-administration with a compound of formula (I) or a pharmaceutically acceptable form thereof). The kit may optionally provide other component such as a buffer and an instruction. The instruction provided in the kit of the disclosure is generally a written instruction on a label or package insert (e.g., a sheet of paper contained in the kit), but a machine-readable instruction (e.g., an instruction carried on a magnetic disk or optical disk) is also acceptable.

In the foregoing first to fourth aspects,

In some embodiments, in the compound of formula (I), $R^2$ is selected from the group consisting of:

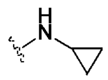 , 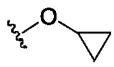 .

In some embodiments, in the compound of formula (I), U is selected from the group consisting of:

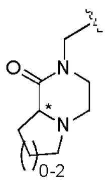 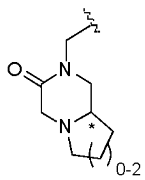 .

In some embodiments, in the compound of formula (I), $R^2$ is:

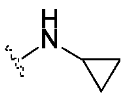 .

In some embodiments, in the compound of formula (I), U is:

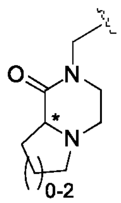 .

In some embodiments, in the compound of formula (I), $R^2$ is:

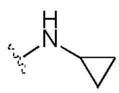 ,

U is:

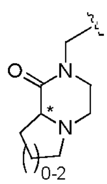 .

In some embodiments, the compound of formula (I) is a compound of Formula (A):

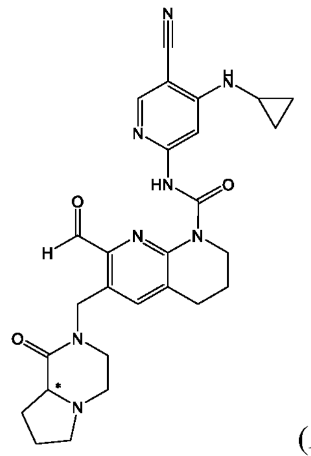

(A).

In some embodiments, the compound of formula (I) is a compound of Formula (B):

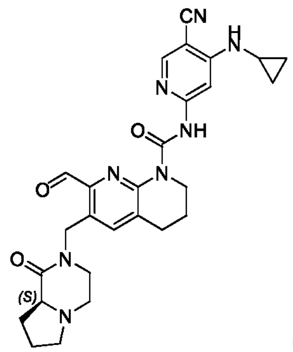

(B).

In some embodiments, the pharmaceutically acceptable form of the compound of formula (I) includes: a solvate, a hydrate, a pharmaceutically acceptable salt, a stereoisomer, a tautomer, an isotopic derivative, a co-crystal, a polymorph, a prodrug, and a metabolite of the compound of formula (I).

In some embodiments, the other anticancer agent is a CDK4/6 inhibitor or a pharmaceutically acceptable salt thereof, such as palbociclib, ribociclib, abemaciclib, trilaciclib, G1T-38, G1T-28, AT-7519, FLX-925, alvocidib, and the like, or a pharmaceutically acceptable salt thereof, preferably palbociclib. In some embodiments, the other anticancer agent is lenvatinib mesylate. In some embodiments, the other anticancer agent is sorafenib tosylate. In some embodiments, the malignant tumor is a solid tumor, such as lung cancer, bladder cancer, breast cancer, gastric cancer, hepatic cancer, salivary gland sarcoma, ovarian cancer, prostate cancer, cervical cancer, epithelial cell cancer, pancreatic cancer, more preferably hepatic cancer; the hepatic cancer is preferably hepatocellular carcinoma, intrahepatic cholangiocarcinoma.

In some embodiments, the malignant tumor is a refractory tumor that is resistant to other therapeutic method. In some embodiments, the malignant tumor is a recurrent tumor after undergoing other treatment method. In some embodiments, the malignant tumor is unresectable. In some embodiments, the malignant tumor is metastatic. In some embodiments, the malignant tumor is advanced. In some embodiments, the malignant tumor is mediated by FGFR4. In some embodiments, the malignant tumor is an aberrant FGFR4 signaling pathway. In some embodiments, the malignant tumor is characterized by FGR19 amplification or FGF19 overexpression. In some embodiments, the malignant tumor is characterized by FGF19 overexpression without statistically significant FGR19 amplification.

In some embodiments, the mass ratio of the compound of formula (I) or a pharmaceutically acceptable form thereof to the other anticancer agent may be 1-30:0.8-80, preferably 1-20:0.8-80, preferably 3-20:0.8-80, preferably 1-30:7.5-12.5, or 1-30:0.8-1.2, or 1-30:40-80, preferably 1-20:7.5-12.5, or 1-20:0.8-1.2, or 1-20:40-80, preferably 3-20:7.5-12.5, or 3-20:0.8-1.2, or 3-20:40-80, preferably 3-12:7.5-12.5, or 3-12:0.8-1.2, or 3-12:40-80, preferably 5:8-50, preferably 5:8, or 5:50.

In some embodiments, a compound of formula (I) or a pharmaceutically acceptable form thereof may be administered simultaneously with or separately from other anticancer agents. The administration may be oral administration, injection administration, topical administration or in vitro administration.

In some embodiments, a compound of formula (I) or a pharmaceutically acceptable form thereof and other

anticancer agent may be included in the same pharmaceutical formulation, or may be separately prepared into a clinically acceptable formulation, and the medicine may be prepared in a combined package, or administered simultaneously or separately in a separate formulation. The clinically acceptable formulation includes oral formulation, injection formulation, topical formulation, external formulation, and the like. In some embodiments, the medicine is in a single-dose dosage form or a divided-dose dosage form.

The therapeutically effective amount or effective amount described in the disclosure refers to an effective dose in pharmacology, i.e., an amount of an active compound sufficient to significantly ameliorate the condition without causing a serious side effect. It may be administered in single dose once daily, in divided doses daily, or at interval. The specific dose and administration frequency should consider a variety of factors such as administration route and patient's health condition, which can be determined by a skilled physician in accordance with conventional skills. There is no specific restriction on the administration route, and a representative administration route includes, but is not limited to: oral, rectal, parenteral (intravenous, intramuscular or subcutaneous) and topical administration.

In some embodiments, a compound of formula (I), such as a compound of formula (B), or a pharmaceutically acceptable form thereof, is administered once or twice or thrice daily. In some embodiments, a compound of formula (I), such as a compound of formula (B), or a pharmaceutically acceptable form thereof, is administered once daily. In some embodiments, up to 300 mg of a compound of formula (I), such as a compound of formula (B), or an equivalent amount of a pharmaceutically acceptable form thereof is administered daily. For example, in some embodiments, 1-1000 mg, preferably 5-500 mg, more preferably 10-300 mg, or 10-270 mg, or 10-240 mg, or 10-210 mg, or 10-200 mg, or 10-180 mg, or 10-150 mg, or 10-120 mg, or 10-90 mg, or 10-60 mg, or 30-300 mg, or 30-270 mg, or 30-240 mg, or 30-210 mg, or 30-200 mg, or 30-180 mg, or 30-150 mg, or 30-120 mg, or 30-90 mg, or 30-60 mg, and further preferably 10 mg, 20 mg, 30 mg, 60 mg, 90 mg, 120 mg, 150 mg, 180 mg, 210 mg, 240 mg, 270 mg, or 300 mg of a compound of formula (I), such as a compound of formula (B), or an equivalent amount of a pharmaceutically acceptable form thereof is administrated daily. In some embodiments, less than 30 mg of a compound of formula (I), such as a compound of formula (B), or an equivalent amount of a pharmaceutically acceptable form thereof is administered orally to a patient once daily. In some embodiments, a compound of formula (I), such as a compound of formula (B), or a pharmaceutically acceptable form thereof is administered in the form of a tablet.

In some embodiments, 75-125 mg of palbociclib or an equivalent amount of a pharmaceutically acceptable salt thereof is administered orally to a patient once daily. In some embodiments, 125 mg of palbociclib or an equivalent amount of a pharmaceutically acceptable salt thereof is administered orally to a patient once daily. In some embodiments, less than 125 mg of palbociclib or an equivalent amount of a pharmaceutically acceptable salt thereof is administered orally to a patient once daily, for example, 100 mg or 75 mg of palbociclib or an equivalent amount of a pharmaceutically acceptable salt thereof is administered orally to a patient once daily.

In some embodiments, a daily dose of lenvatinib or an equivalent amount of a pharmaceutically acceptable salt thereof is 8 mg-12 mg. In some embodiments, a daily dose of lenvatinib or an equivalent amount of a pharmaceutically acceptable salt thereof is 8 mg. For example, a daily dose of lenvatinib or an equivalent amount of a pharmaceutically acceptable salt thereof is 12 mg.

In some embodiments, a daily dose of sorafenib or an equivalent amount of a pharmaceutically acceptable salt thereof is 0.4 g-0.8 g. In some embodiments, a daily dose of sorafenib or an equivalent amount of a pharmaceutically acceptable salt thereof is 0.4 g, e.g., administrated once daily, 0.4 g each time. In some embodiments, a daily dose of sorafenib or an equivalent amount of a pharmaceutically acceptable salt thereof is 0.8 g, e.g., administrated twice daily, 0.4 g each time.

## DESCRIPTION OF DRAWINGS

Figure 1: a graph of changes in the volume of a xenograft tumor in tumor-bearing mice treated with a compound of formula (B) combined with palbociclib or lenvatinib mesylate.

Figure 2: a graph of changes in the volume of a xenograft tumor in tumor-bearing mice treated with a compound of formula (B) combined with sorafenib tosylate or palbociclib.

## DETAILED DESCRIPTION OF INVENTION

### Definitions and Descriptions

As used herein, the term "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, and

commensurate with a reasonable benefit/risk ratio. Unless defined otherwise, the term "pharmaceutically acceptable salt" or "pharmaceutical salt" used herein refers to salts which are, within the scope of sound medical judgment, suitable for use in contact with the tissues of mammals (especially human beings) without excessive toxicity, irritation, allergic response, etc. and commensurate with a reasonable benefit/risk ratio, for example, pharmaceutically acceptable salts of amines, carboxylic acids and other types of compounds are well known in the art to which they belong. The salts can be prepared in situ during the final isolation and purification of the compound of the disclosure or separately by reacting the free base or free acid with a suitable reagent.

Unless defined otherwise, the term "isotope derivative" refers to that the compound of the disclosure can exist in isotope-labeled or isotope-enriched form containing one or more atoms which have atomic mass or mass number different from the atomic mass or mass number of the atom most abundantly found in nature. Isotopes can be radioactive or non-radioactive isotopes. The isotopes commonly used for isotope labelling are: hydrogen isotope, $^{2}H$ and $^{3}H$; carbon isotope: $^{13}C$ and $^{14}C$; chlorine isotope: $^{35}C1$ and $^{37}Cl$; fluorine isotope: $^{18}F$; iodine isotope: $^{123}I$ and $^{125}I$; nitrogen isotope: $^{13}N$ and $^{15}N$; oxygen isotope, $^{15}O$, $^{17}O$ and $^{18}O$; and sulfur isotope $^{35}S$. These isotope-labeled compounds can be used to study the distribution of pharmaceutical molecules in tissues. In particular, $^{3}H$ and $^{13}C$ are more widely used due to their ease of labeling and detection. The substitution with certain heavy isotopes, such as deuterium ($^{2}H$), can enhance metabolic stability and prolong half-life, thereby achieving the purpose of reducing dose and providing therapeutic advantages. An isotope-labeled compound is generally synthesized starting from a labeled starting material and using known synthetic techniques in the same way as synthesizing a non-isotope-labeled compound.

Unless otherwise specified, the term "solvate" refers to a physical association of a compound of the disclosure with one or more solvent molecules, whether organic or inorganic. This physical association involves hydrogen bonding. In certain instances, the solvate will be capable of isolation, for example when one or more solvent molecules are incorporated in the crystal lattice of the crystalline solid. The solvent molecules in the solvate may be present in a regular arrangement and/or a non-ordered arrangement. The solvate may comprise either a stoichiometric or nonstoichiometric amount of the solvent molecules. The solvate may be a solvate of the disclosed compound or a pharmaceutically acceptable salt thereof. "Solvate" encompasses both solution-phase and isolable solvates. Exemplary solvate includes, but is not limited to, hydrate, ethanolate, methanolate, and isopropanolate. When the solvent is water, this solvate is a "hydrate". Pharmaceutically acceptable solvates and hydrates are, for example, complexes including 1 to about 100, or 1 to about 10, or 1 to about 2, 3 or 4 solvent or water molecules. It is to be understood that the term "compound" as used herein includes this compound, the solvates of this compound, and mixtures thereof. Solvation methods are well known in the art.

Unless otherwise specified, the term "stereoisomers" refers to compounds having identical chemical constitution, but differ in the arrangement of their atoms or groups in space. Stereoisomers include enantiomers, diastereomers, conformational isomers (rotational isomers), geometrical isomers (cis/trans isomers), and blocking isomers. The mixture of any stereoisomers may be separated into pure or substantially pure geometric isomers, enantiomers, diastereomers, on the basis of differences in the physicochemical properties of the components, e.g., by chromatography and/or stepwise crystallization. Unless otherwise specified, the term "tautomeric isomers" refer to structural isomers having different energies that can be transformed to each other through low-energy barriers. If tautomerism is possible (e.g., in solution), chemical equilibrium of the tautomeric isomers can be achieved. For example, proton tautomer (also known as proton transfer tautomer) involves interconversions by proton migration, such as keto-enol isomerization and imino-enamine isomerization. Valence tautomer involves interconversion by the recombination of some bonding electrons.

Unless otherwise indicated, the structural formulae described in the disclosure include all isomeric forms (e.g., enantiomeric isomerism, diastereoisomerism, and geometric isomerism (or conformational isomerism)): e.g., (R) and (S) conformations for an asymmetric center, (Z) and (E) isomers for double-bond, and (Z) and (E) conformational isomers. Thus, individual stereochemical isomers of the compounds of the disclosure or mixtures of enantiomers, diastereomers, or geometrical isomers (or conformational isomers) thereof are within the scope of the disclosure.

"Enantiomeric isomers" or "enantiomers" are a pair of stereoisomers that are non-overlapping mirror images of each other. A mixture of a pair of enantiomers in any ratio may be referred to as a "racemic" mixture. The term "($\pm$)" is used to designate a racemic mixture where appropriate. "Diastereoisomers" or "diastereomers" refer to stereoisomers that have at least two asymmetric atoms, but which are not mirror-images of each other. The absolute stereochemistry can be specified according to the Cahn-Ingold-Prelog R-S system. When the compounds are enantiomers, the stereochemistry on each chiral carbon can be specified by R or S. The isolated compound of unknown absolute configuration can be designated as (+) or (-) according to the direction in which they rotate plane polarized light (right or left) at the wavelength of the sodium D line. Certain compounds described herein contain one or more asymmetric centers and may therefore produce enantiomers, diastereomers and other stereoisomeric forms that may be designated as (R)- or (S)- according to the absolute stereochemistry at each asymmetric atom. The chemical entities, pharmaceutical compositions and methods of the disclose are intended to include all such possible isomers, including racemic mixtures, optically substantially pure forms and intermediate mixtures. For example, the rotary (R)- and (S)- isomers can be prepared by using chiral synthons or chiral reagents or isolated by using conventional techniques.

In some embodiments, isomers/enantiomers may be provided substantially free of the corresponding enantiomer, and may also be referred to as "optically enriched", "enantiomerically enriched", "enantiomerically pure", and "non-racemic", as used interchangeably herein. These terms refer to compositions in which the amount of one enantiomer is greater than the amount of that enantiomer in a control mixture of the racemic composition (e.g., greater than 1:1 by weight). For example, an enantiomerically enriched product of S enantiomer refers to a compound product having greater than about 50% by weight, such as at least about 75% by weight, further such as at least about 80% by weight of the S enantiomer, with respect to the total weight of the product (e.g., the total weight of S and R isomers). In some embodiments, the enrichment may be much greater than about 80% by weight, providing a "substantially enantiomerically enriched", "substantially enantiomerically pure" or "substantially non-racemic" product, which refers to a compound product having at least about 85% by weight, such as at least about 90% by weight, further such as at least about 95% by weight, of one enantiomer with respect to the total weight of the product. In certain embodiments, the compound provided herein is made up of at least about 90% by weight of one enantiomer. In other embodiments, the compound is made up of at least about 95%, about 98%, or about 99% by weight of one enantiomer.

In some embodiments, the compound is a racemic mixture of (S)- and (R)-isomers. In other embodiments, provided herein is a mixture of compounds wherein individual compounds of the mixture exist predominately in (S)- or (R)-isomeric configuration. For example, in some embodiments, the compound mixture has (S)-enantiomeric excess of greater than 10%, greater than about 20%, greater than about 30%, greater than about 40%, greater than about 50%, greater than about 55%, greater than about 60%, greater than about 65%, greater than about 70%, greater than about 75%, greater than about 80%, greater than about 85%, greater than about 90%, greater than about 95%, greater than about 96%, greater than about 97%, greater than about 98%, or greater than about 99%. In some embodiments, the compound mixture has (S)-enantiomeric excess of about 55%, about 60%, about 65%, about 70%, about 75%, about 80%, about 85%, about 90%, about 95%, about 96%, about 97%, about 98%, about 99%, or about 99.5% or greater. In some embodiments, the compound mixture has (S)-enantiomeric excess of about 55% to about 99.5%, about 60% to about 99.5%, about 65% to about 99.5%, about 70% to about 99.5%, about 75% to about 99.5%, about 80% to about 99.5%, about 85% to about 99.5%, about 90% to about 99.5%, about 95% to about 99.5%, about 96% to about 99.5%, about 97% to about 99.5%, about 98% to about 99.5%, or about 99% to about 99.5%, or greater than about 99.5%.

Unless otherwise specified, the term "co-crystalline" or "co-crystal" is used to describe a situation in which the neutral molecular component is present within the crystalline compound in a well-defined stoichiometric ratio. Preparation of a pharmaceutical co-crystal enables to change the crystalline form of the active pharmaceutical ingredient, which in turn can alter its physicochemical properties without compromising its desired biological activity (see Pharmaceutical Salts and Co-crystals, Johan Wouters and Luc Quéré (Eds.), RSC Publishing, 2012).

Unless otherwise specified, the term "polycrystalline" or "polymorph" refers to a different arrangement of chemical-medicine molecules, generally expressed as the form in which the pharmaceutical raw material exists in solid state. One medicine may exist as a material having a plurality of crystalline forms, and different crystalline forms of the same medicine may have different dissolution and absorption in the body, thereby affecting the dissolution and release of the formulation. In certain embodiments, "polycrystalline" or "polymorph" as used herein is also intended to include all crystalline and amorphous forms of the compound or pharmaceutically acceptable salt thereof, including, for example, crystalline forms, polymorphs, pseudopolymorphs, solvates, hydrates, co-crystals, non-solvated polymorphs (including anhydrous), conformational polymorphs, reciprocal isomeric forms, disordered crystalline forms, amorphous forms, and mixtures thereof, unless a specific crystalline or amorphous form is mentioned.

Unless otherwise specified, the term "metabolite" refers to a product obtained in the body by metabolism of a particular compound or a pharmaceutically acceptable salt thereof. A metabolite of a compound can be identified by techniques well known in the art and its activity can be characterized by assays as described in the disclosure. Such products can be obtained by subjecting the administered compound to oxidation, reduction, hydrolysis, amidation, deamidation, esterification, degreasing, enzymatic cleavage, and the like. Accordingly, the disclosure includes a metabolite of a compound, including a metabolite produced by sufficiently contacting a compound of the disclosure with mammals for a period of time.

Unless otherwise specified, the term "prodrug" refers to a drug that is converted into a parent drug in vivo. Prodrugs are generally useful, which may improve some of the identified, undesirable physical or biological properties. Physical properties are generally related solubility (excessive or insufficient lipid or water solubility) or stability, while problematic biological properties include too rapid metabolism or poor bioavailability, which may themselves be related to physicochemical properties. For example, they may be bioavailable by oral administration, while the parent is not. The solubility of the prodrug in the pharmaceutical composition is also improved compared to the parent drug. One example of prodrug (but not limited to) may be any of the compounds herein administered as an ester ("prodrug") to facilitate delivery across cell membranes (water solubility is detrimental to migratory properties, but water solubility is beneficial once coming into a cell), which is subsequently metabolically hydrolyzed to a carboxylic acid, i.e., the active entity. Another example of prodrug may be a short peptide (polyamino acid) bound to an acid group in which the peptide is metabolized to show an active moiety.

As used herein, "combined" administration or "combination" administration refers to that two (or more) different

therapies are delivered to a subject during the course of the subject's affliction with the condition, e.g., two or more drugs are delivered to the subject after the subject has been diagnosed with a condition and before the condition has been cured or eliminated or the therapy has ceased for other reasons. In some embodiments, when delivery of the second treatment begins, delivery of the first treatment is still ongoing, so there is overlap in terms of administration. This situation is sometimes referred to as "simultaneous delivery" or "concurrent delivery" herein. In other embodiments, delivery of one treatment has ended before delivery of another treatment begins. In some embodiments of either case, the treatment is more effective due to combined administration. For example, the second treatment is more effective, e.g., an equivalent effect is obtained with less of the second treatment, or the second treatment reduces symptoms to a greater extent, than would be obtained if the second treatment were administered in the absence of the first treatment, or the analogous situation is obtained with the first treatment. In some embodiments, the above delivery is such that the reduction in a symptom or other parameter related to the disorder is greater than what would be observed with one treatment delivered in the absence of the other. The effect of the two (or more) treatments can be partially additive, wholly additive, or greater than additive. The delivery can be such that an effect of the first treatment delivered is still detectable when the second treatment is delivered.

As used herein, the term "single agent" refers to a single carrier or vehicle formulated to deliver an effective amount of both therapeutic agents to a patient. The single vehicle is designed to deliver an effective amount of each of the agents, along with any of pharmaceutically acceptable carriers or excipients. In some embodiments, the vehicle is a tablet, a capsule, a pill, or a patch.

The term "unit dose" is used herein to mean that both agents are administered together in one dosage form to the patient being treated. In some embodiments, the unit dose is a single formulation. In certain embodiments, the unit dose comprises one or more vehicles such that each vehicle contains an effective amount of at least one of agents (e.g., a compound of formula (I) or a CDK inhibitor or a pharmaceutically acceptable salt thereof, lenvatinib or a pharmaceutically acceptable salt thereof, sorafenib or a pharmaceutically acceptable salt thereof) with a pharmaceutically acceptable carrier and excipient. In some embodiments, the unit dose is one or more tablets, capsules, pills, or patches that are administered simultaneously to the patient.

The term "pharmaceutically acceptable excipient" or "pharmaceutical excipient" may be selected from carriers, binders, suspending agents, glidants, flavoring agents, disintegrants, dispersing agents, surfactants, lubricants, colorants, diluents, solubilizers, moistening agents, stabilizers, penetration enhancers, defoamers, antioxidants, preservatives, solvents or combinations thereof commonly used in the art.

The term "dosage range" refers to the upper and lower limits of acceptable changes in the amount of the specified agent. In general, any amount of the agent within the specified range may be administered to a patient being treated.

The term "treating" means alleviating, reducing or alleviating at least one symptom of a disease in a subject. For example, for a malignant tumor disease, the term "treating" may means arresting, delaying the onset of a disease (i.e., the period prior to clinical manifestation of a disease or a symptom thereof) and/or reducing the risk of developing or worsening a symptom of a disease. The term, when used in conjunction with a disease such as cancer, includes, but is not limited to, one or more of the group consisting of: arresting growth of the cancer; causing the cancer to shrink in weight or volume; prolonging the expected survival time of a patient; inhibiting tumor growth; reducing tumor mass; reducing size or number of metastatic lesions; inhibiting the development of new metastatic lesions; prolonging survival; prolonging progression-free survival; prolonging time to progression; and/or improving quality of life.

The terms "treatment", "alleviation", and "amelioration" are used interchangeably herein. These terms refer to methods for obtaining beneficial or desired results including, but not limited to, therapeutic benefit and/or prophylactic benefit. Therapeutic benefit refers to eradication or amelioration of the underlying disorder being treated. Eradication or amelioration of one or more physiological symptoms associated with the underlying disorder also achieves therapeutic benefits such that amelioration is observed in the patient, although the patient may still be afflicted with the underlying disorder. With regard to prophylactic benefit, the pharmaceutical composition may be administered to a patient at risk of developing a particular disease or to a patient reporting one or more physiological symptoms of the disease, even though the disease may not have been diagnosed yet. In one embodiment, these terms also refer to partial or complete inhibition or alleviation of a condition in a subject. In one embodiment, these terms refer to an action taken to alleviate the severity of the condition or delay or slow the progression of the condition when a patient is suffering from or diagnosed with the condition. Treatment need not to result in complete cure of the condition; this term includes partial inhibition or alleviation of the condition. Treatment is intended to include prevention or prophylaxis.

The term "subject" or "patient" is intended to include an animal suffering from a malignant tumor disease. Examples of subject or patient include mammals, e.g., humans, dogs, cows, horses, pigs, sheep, goats, cats, mice, rabbits, rats, and transgenic non-human animals. In certain embodiments, the subject is a human, e.g., a human suffering from, at risk of suffering from, or potentially capable of suffering from a malignant tumor disease.

The term "about" or "approximately" generally means within 20%, more preferably within 10%, and most preferably within 5% of a given value or range. Alternatively, the term "about" means within 1 logarithm (i.e., an order of magnitude) of a given value, preferably within 2 times.

The term "enhanced effect" refers to that the effect of two agents administered together provides greater or improved results than that of one single agent administered alone without co-administration with the other agent. Administration of the agents together may provide an enhanced effect when they are administered simultaneously or sequentially. Sequential administration of agents includes administration at intervals of a few seconds, minutes, hours, or days. Co-administration of agents may provide an enhanced effect when the agents are administered as part of a single formulation or when the agents are administered as separate formulations. Examples of agents that can be administered together are a compound of formula (I) and a CDK inhibitor or a pharmaceutically acceptable salt thereof, lenvatinib or a pharmaceutically acceptable salt thereof, sorafenib or a pharmaceutically acceptable salt thereof, or any combination of the foregoing.

The term "expanded" means that additional copies of genes or chromosomal segments that confer growth or survival advantages are produced in cancer cells. One skilled in the art can measure the copy number of genes or chromosomal segments by using conventional techniques in the art, such as fluorescence in situ hybridization (FISH) comparative genomic hybridization, and high resolution array-based assay which is based on: array comparative genomic hybridization (or aCGH), SNP array techniques, and high resolution microarrays including copy number probes and SNPs, as well as whole genome sequencing (WGS) or whole exome DNA sequencing (WES) using next generation sequencing (NGS) technique.

The term "FGFR4" or "FGFR4 protein" refers to any form of FGFR4 protein, including wild-type and all variant forms (including but not limited to mutant forms and splice variants). FGFR4 protein is a product of FGFR4 gene and therefore includes any protein encoded by any form of FGFR4 gene including any distortion (e.g., point mutation, insertion deletion, translocation fusion, and focal amplification). The term "overexpression" means that the yield of a gene product in sample is higher than that observed in a control sample population (e.g., normal tissue). If a gene product is generally not produced in a control sample, overexpression includes expression. The yield of a gene product can be measured using conventional techniques in the art, such as immunohistochemistry. In one aspect, FGF19 gene product overexpression means FGF19 protein expression ≥1%.

The term "therapeutic effect" refers to a beneficial local or systemic effect in an animal such as mammal (e.g., human) caused by the administration of a compound or composition of the disclosure. The phrase "therapeutically effective amount" refers to an amount of a compound or composition of the disclosure that is effective in treating a disease or condition at a reasonable benefit/risk ratio. The therapeutically effective amount of the compound or composition will vary depending on the subject and the disease or disorder being treated, the weight and age of the subject, the severity of the disease or disorder, the mode of administration, and the like, which is readily determined by one skilled in the art.

The term "combination therapy" refers to a dose regimen of administrating at least two different compounds, such as a compound of formula (I) and a CDK inhibitor or a pharmaceutically acceptable salt thereof, lenvatinib or a pharmaceutically acceptable salt thereof, sorafenib or a pharmaceutically acceptable salt thereof, or any combination of the foregoing, to a patient. The at least two different compounds may be administered simultaneously or at different times within one day. The dose regimens of the at least two compounds may, but need not to, overlap.

The term "total daily dose" or "daily administered dose" refers to the amount of a compound administered to a subject within a time window of 24 hours.

The term "co-administration" means exposing a subject to two or more treatment regimens (e.g., two or more compounds) simultaneously. In some embodiments, two or more compounds may be administered simultaneously; in some embodiments, two or more compounds can be administered sequentially (in a completely non-overlapping dose regimen); in some embodiments, two or more compounds may be administered in a partially overlapping dose regimen. In some embodiments, "administration" of the combination therapy may involve administering one or more compounds to a subject who has received (one or more) other compound(s). For clarity, combination therapy does not require the compounds to be administered together (or even not necessarily to be administered at the same time) in the form of a single composition, but in some embodiments, two or more compounds may be administered together in the form of a single composition. In some embodiments, compounds to be co-administered are in separate dosage forms, but packaged together (e.g., in blister packages or other kits) to facilitate co-administration thereof.

In this context, when referring to "A in combination with B for the treatment of a disease", "A and B combined for the treatment of a disease" or "A in combination with B for the preparation of a drug for the treatment of a disease" or the like, it is generally meant that A and B may produce a synergistic effect in the treatment of a disease, i.e., the combined therapeutic effect of A and B is superior to the individual therapeutic effect of A or B, or the combined side effect/adverse reaction of A and B is lower than the individual side effect/adverse reaction of A or B, including but not limited to, A may enhance the therapeutic effect of an equivalent dose of B, such that the combined therapeutic effect of A and B may be superior to the sum of the individual therapeutic effects of A and B; A may reduce the dose of B while producing equivalent efficacy, thereby reducing the side effects/adverse reactions that may occur with high doses of B; A may directly reduce or avoid side effects/adverse reactions that B may cause; etc.

It will be understood that the examples or embodiments described herein are to be considered in a descriptive sense only, and not for purposes of limitation. Descriptions of features in each example or embodiment should be

considered as being applicable to other similar features in other examples or embodiments. It will be understood by one of ordinary skill in the art that various changes in form and detail can be made without departing from the spirit and scope as defined by the claims.

## Examples

The disclosure is further illustrated below in connection with specific examples. It will be understood that these examples are merely illustrative of the disclosure and not intended to limit the scope of the disclosure. Experimental methods which are not specified in the following examples are generally according to conventional conditions, or according to conditions suggested by the manufacturer. The raw materials and reagent used, not specifically noted, are commercially available. The compound of formula (I) of the disclosure can be prepared by referring to the method disclosed in Chinese patent application CN108948004A.

Preparation Example: Preparation of Compound of Formula (B)

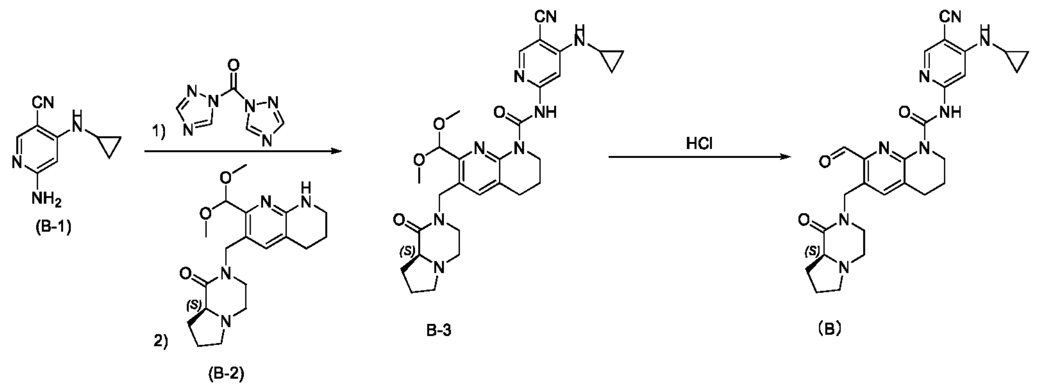


(B-1) (B-2) B-3 (B)

Compound B-1 (6 g, 34.5 mmol) and N,N-carbonylbis(1,2,4-triazole) (17 g, 103.4 mmol) were dissolved in dried N,N-dimethylformamide (60 mL), the mixture solution was stirred at room temperature for 2 hours, and then compound B-2 (8.7 g, 24.1 mmol) (prepared using S configuration 10a with reference to example 10 of WO2017202390A1) was added thereto. The reaction system was stirred at room temperature overnight. After completion of reaction monitored by HPLC, the reaction solution was quenched with 10% of lithium chloride solution, extracted three times with ethyl acetate. The organic layers were combined and washed with saturated brine, then dried over anhydrous sodium sulfate. After concentration, the obtained crude product was separated and purified by column chromatography (dichloromethane/-methanol =25/1-20/1), to give compound B-3 as white solid (1.5 g, 11% yield).

Compound B-3 (1.5 g, 2.7 mmol) was dissolved in tetrahydrofuran (30 mL), and then HCl (2 M, aqueous solution) (20 mL) was added dropwise to the solution at room temperature. The reaction solution was stirred at room temperature for 3 hours. The reaction solution was poured into an appropriate amount of saturated sodium bicarbonate solution, extracted three times with dichloromethane. The organic layers were combined and washed with saturated brine, then dried over anhydrous sodium sulfate. After concentration, the crude product obtained was separated and purified by column chromatography (dichloromethane/methanol =20/1), to give the compound of formula (B) as off-white solid (0.44 g, 32% yield). $^1$HNMR(500 MHz, $CDCl_3$) $\delta$ 13.56 (s, 1H), 10.17 (s, 1H), 8.11 (s, 1H), 7.85 (s, 1H), 7.57 (s, 1H), 5.21 (s, 1H), 5.00 (dd, J = 46.1, 15.7 Hz, 2H), 4.07-3.96 (m, 2H), 3.45 (ddd, J = 12.3, 9.6, 4.8 Hz, 1H), 3.23 (ddd, J = 16.8, 10.7, 6.3 Hz, 2H), 2.98 (dt, J = 12.2, 4.3 Hz, 1H), 2.91 (ddd, J = 9.5, 7.6, 4.8 Hz, 1H), 2.85 (t, J = 6.3 Hz, 2H), 2.79-2.69 (m, 1H), 2.61-2.51 (m, 2H), 2.23-2.14 (m, 1H), 2.00-1.94 (m, 2H), 1.94-1.69 (m, 3H), 0.93-0.85 (m, 2H), 0.63-0.55 (m, 2H). MS 515.20 $[M+H]^+$.

### Example 1: In Vitro Pharmacological Test of Compound of Formula (B)

1. FGFR4 kinase activity inhibition experiment

FGFR4 protein kinase activity was determined using Caliper mobility shift assay. The compound was dissolved in DMSO and then diluted with kinase buffer, 5 $\mu$L of 5-fold final reaction concentration of compound (10% DMSO) was added to a 384-well plate. 10 $\mu$L of 2.5-fold enzyme (FGFR4) solution was added and incubated at room temperature for 10 minutes, followed by 10 $\mu$L of 2.5-fold substrate (FAM-labeled peptide and ATP) solution. After incubation at 28°C for 30-60 minutes, 25 $\mu$L of stop solution was added to stop the reaction. Conversion data was read on Caliper EZ Reader II (Caliper Life Sciences). The conversion rate was converted to inhibition rate (% inhibition = (max - sample conversion rate)/(max-min)*100), wherein max refers to the conversion rate of the DMSO control, and min refers to the conversion rate of enzyme-free control. Curve was plotted with compound concentration and inhibition rate on the horizontal and vertical coordinates

respectively, and the curve was fitted and $IC_{50}$ was calculated using XLFit excel add-in version 4.3.1 software.

The results indicate that the FGFR4 kinase activity inhibition ($IC_{50}$, nM) of the compound of formula (B) of the disclosure is <5 nM.

2. Huh-7 Tumor Cell Proliferation Inhibition Assay

Huh-7 cell suspension was adjusted to $5 \times 10^4$/ml or $2 \times 10^4$/ml with DMEM+2 mM Glutamine+10% FBS medium. 100 μL of cell suspension was added per well to a 96-well cell culture plate, to give a final cell concentration of 5000 cells/well (72 hours) or 2000 cells/well (168 hours). The compound to be tested (compound of formula (B)) was dissolved in DMSO to give 10 mmol of stock solution. 200X final concentration of compound was prepared with stock solution and DMSO, and 3X serial gradient dilutions were prepared, then diluted 20-fold each with medium. Finally, 10 μL of the corresponding 10-fold solution was added per well for each cell, wherein each drug concentration was added in one well. The final treatment concentration of each compound was 3000 nM, 1000 nM, 333.3 nM, 111.1 nM, 37.04 nM, 12.35 nM, 4.12 nM, 1.37 nM, respectively, and the final concentration of DMSO per well is 0.5%. The plate was placed in a 37°C, 5% $CO_2$ incubator for 72 or 168 hours. After 72 or 168 hours of drug treatment, according to CTG instructions (CellTiter Glo), 100 μL of CTG solution that had been previously thawed and equilibrated to room temperature was added per well, mixed for 2 minutes using a microplate shaker, placed at room temperature for 10 minutes, and then chemiluminescent signal value was measured with EnSpire plate reader. Cell survival was calculated using formula: $(V_{sample}-V_{blank})/(V_{vehicle\ control}-V_{blank}) \times 100\%$, wherein $V_{sample}$ is the reading of the drug treatment group, $V_{vehicle\ control}$ is the average of the solvent control group, and $V_{blank}$ is the average of the blank control wells. Using GraphPad Prism 5.0 software, a sigmoidal dose-survival curve was plotted and $IC_{50}$ value was calculated using a non-linear regression model.

The results indicate that the Huh-7 tumor cell proliferation inhibition ($IC_{50}$, nM) of the compound of formula (B) of the disclosure is <10 nM.

**Example 2: Pharmacodynamical Assay of Compound of Formula (B) in Combination with Lenvatinib Mesylate or Palbociclib on NU/NU Mice Hep3B (Human Hepatocarcinoma Cell Line) Xenograft Model.**

**1. Test Animals**

42 NU/NU mouse, female, 42-48 days old, weighing 22-26 g.

**2. Test Objective**

To investigate the efficacy of compound of formula (B) in combination with lenvatinib mesylate or palbociclib on Hep3B xenograft model.

**3. Drug**

Compound of formula (B), lenvatinib mesylate, palbociclib.

Solvent: Compound of formula (B): 0.5% methylcellulose aqueous solution containing 0.4% Tween 80;
Lenvatinib Mesylate/Palbociclib: a solution of castor oil, anhydrous ethanol and ultrapure water in a volume ratio of 1:1:6.

**4. Test Method**

4.1 Model Preparation

Hep3B cells were resuscitated and passaged in vitro to the desired number of cells. The cells were counted under a microscope, and diluted with serum-free medium and matrix gel (1:1) to adjust the number of tumor cells to approximately $5 \times 10^7$ cells/mL. The cell suspension was placed in an ice bath.

Hep3B cell suspension was withdrawn with a sterile syringe for inoculation into subcutaneous tissue in the axilla of the forelimb of NU/NU mice, with a volume of 0.1 mL/per mice, containing approximately $5.0 \times 10^6$ tumor cells, to prepare NU/NU mice Hep3B xenogratf model.

4.2 Method of Administration

Single drug group (compound of formula (B), lenvatinib mesylate, palbociclib) and combination administrated

group (compound of formula (B) + lenvatinib mesylate, compound of formula (B) + palbociclib), administrated once daily by gavage; volume of administration was 10 mL/kg. In the vehicle group, the solvents were administered at the same frequency and volume for 14 consecutive days.

4.3 Observation Criteria and Evaluation Criteria

(1) General state observation: all animals were observed once a day during the test period and the abnormalities in various parts of the body and the behavior change situations were recorded.
(2) Body weight: all animals were weighed once before testing and the appropriate body weight of animals were selected for testing. Animals were weighed once a day at a fixed time after initiation of administration for weight monitoring, and body weight statistics were consistent with tumor volume statistics.
(3) Death and moribund: time of death was recorded for dead animals, moribund animals were observed more frequently, and time of death was determined.
(4) Tumor Evaluation

After animal grouping, tumor length and short diameters were measured twice per week.

① Tumor volume:

$$V = 1/2 \times a \times b^2$$

② Relative tumor volume:

$$RTV = \frac{TV_{nd}}{TV_{0d}}$$

③ Relative tumor volume proliferation rate:

$$T/C\% = \frac{\text{Administrated Group } RTV_{xnd}}{\text{Solvent Group (Model Group) } RTV_{xnd}} \times 100\%$$

④ Tumor growth inhibition rate:

$$TGI\% = \left[1 - \frac{TV_{Xn} - TV_{X0}}{TV_{Mn} - TV_{M0}}\right] \times 100\%$$

Note: V: Tumor volume

a: tumor length; b: tumor width; RTV: relative tumor volume; $TV_{nd}$: tumor volume on day n; $TV_{0d}$: tumor volume on day 0; $RTV_{xnd}$: mean relative tumor volume on day n; $TV_{Xn}$: mean tumor volume on day n in the administrated group; $TV_{X0}$: mean tumor volume on day 0 in the administrated group; $TV_{Mn}$: mean tumor volume on day n in the solvent (model) group; $TV_{M0}$: mean tumor volume on day 0 in the solvent (model) group.

(5) Tumor weight

At the end of the test, the animals were sacrificed by cervical dislocation, and a tumor was stripped and weighed.
Difference in tumor weight between groups: tumor weight inhibition rate% = (1- tumor weight of administrated group/tumor weight of solvent (model) group) ×100%

**5 Statistical Method**

Data were processed using SPSS 19.0 statistical software. Repeated Measure process analyzed tumor volume changes between groups with multiple measurements over time. Multivariate process compared differences in tumor

volume between groups at each measurement. Difference in tumor weight between groups was analyzed by LSD method of One-way ANOVA.

**6 Results**

**6.1 Effect on Tumor Growth**

The results showed that: compared to the vehicle (model) group at corresponding time points, d5-d14, compound of formula (B) 5 mg/kg qd + palbociclib 50 mg/kg qd significantly inhibited tumor volume growth (P≤0.05 or P≤0.001); d8-d14, compound of formula (B) 5 mg/kg qd + lenvatinib mesylate 8 mg/kg qd significantly inhibited tumor volume growth (P≤0.01 or P≤0.001); d12-14, compound of formula (B) 5 mg/kg qd + lenvatinib mesylate 8 mg/kg qd both significantly inhibited tumor volume growth (P≤0.01 or P≤0.001).

At test endpoint (d14), tumor growth inhibition rates (TGI%) of compound of formula (B) 5 mg/kg qd group, lenvatinib mesylate 8 mg/kg qd group, palbociclib 50 mg/kg qd group, compound of formula (B) 5 mg/kg qd + lenvatinib mesylate 8 mg/kg qd group, compound of formula (B) 5 mg/kg qd + palbociclib 50 mg/kg qd group were 49.1%, 57.4%, 11.0%, 72.8% and 88.3%, respectively, and relative tumor volume proliferation rates (T/C%) were 55.8%, 50.9%, 95.8%, 36.2% and 24.2%, respectively.

The results showed that, at the test dose, monotherapy with palbociclib failed to inhibit the growth of xenograft tumor (P > 0.05), while compound of formula (B) alone (P≤0.01), lenvatinib mesylate alone (P≤0.01), the combination of compound of formula (B) and lenvatinib mesylate or palbociclib (P≤0.001) significantly inhibited the growth of the xenograft tumor respectively, wherein the effect of inhibiting tumor of the combination of the compound of formula (B) and lenvatinib mesylate or palbociclib was significantly superior to that of monotherapy. Tumor observations for each group are shown in Table 1 and Figure 1 in detail. In addition, when the compound of formula (B) is administered in combination with lenvatinib mesylate or palbociclib, the change rate in animal weight thereof is significantly less than that of vehicle group and monotherapy group.

**Table 1** Effect of Combined Administration of Compound of Formula (B) and Lenvatinib Mesylate or Palbociclib on Tumor Growth (Mean ± SD, n = 7)

| Group | Dose | Number of Animals (Survived/Total) | Change Rate in Weight (%) | TV ($mm^3$) d14 | T/C d14 (%) | TGI d14 (%) |
|---|---|---|---|---|---|---|
| Vehicle Group | --- | 7/7 | -4.8 | 1,047.5±380.3 | --- | --- |
| Compound of formula (B) | 5 mg/kg qd | 7/7 | -4.5 | 607.8±277.1** | 55.8 | 49.1 |
| Lenvatinib Mesylate | 8 mg/kg qd | 7/7 | -5.8 | 533.1±212.6** | 50.9 | 57.4 |
| Palbociclib | 50 mg/kg qd | 7/7 | 0.8 | 948.8±361.2 | 95.8 | 11.0 |
| Compound of formula (B) + Lenvatinib Mesylate | 5 mg/kg qd /8 mg/kg qd | 7/7 | 0.4 | 395.5±211.4*** | 36.2 | 72.8 |
| Compound of formula (B) + Palbociclib | 5 mg/kg qd /50 mg/kg qd | 7/7 | 0.0 | 257.0 ±139.7*** | 24.2 | 88.3 |
| Note: compared to the vehicle group, **P≤0.01, ***P≤0.001; qd: administrated once daily (same below). | | | | | | |

**6.2 Tumor Weight**

Compared with the vehicle group, the tumor weights of the compound of formula (B) 5 mg/kg qd group, lenvatinib mesylate 8 mg/kg qd group, the compound of formula (B) 5 mg/kg qd + lenvatinib mesylate 8 mg/kg qd group, and the compound of formula (B) 5 mg/kg qd + palbociclib 50 mg/kg qd group were all significantly reduced (P≤0.01), and the tumor weight inhibition rates (%) were 43.8%, 58.0%, 77.9%, and 89.2%, respectively; the tumor weight of palbociclib 50 mg/kg qd group was not statistically different from that of solvent group (P > 0.05), and the tumor weight inhibition rate (%) was only 2.1%.

The results showed that, at the test dose, palbociclib monotherapy did not inhibit the growth of tumor (P > 0.05),

while compound of formula (B) alone (P≤0.01), lenvatinib mesylate alone (P≤0.001) and the combination of compound of formula (B) and lenvatinib mesylate or palbociclib (P≤0.001) significantly inhibited the growth of the xenograft tumor respectively, wherein the effect of inhibiting tumor of the combination of a compound of formula (B) and lenvatinib mesylate or palbociclib was significantly superior to that of single drug. See Table 2 for details.

**Table 2** Effect of Combined Administration of Compound of Formula (B) and Lenvatinib Mesylate or Palbociclib on Tumor Weight (Mean ± SD, n = 7)

| Group | Dose | Number of Animals (Survived/Total) | Tumor Weight (g) | Tumor Weight Inhibition Rate (%) |
|---|---|---|---|---|
| Vehicle Group | --- | 7/7 | 1.3320±0.5104 | --- |
| Compound of formula (B) | 5 mg/kg qd | 7/7 | 0.7481±0.3692** | 43.8 |
| Lenvatinib Mesylate | 8 mg/kg qd | 7/7 | 0.5600±0.1923*** | 58.0 |
| Palbociclib | 50 mg/kg qd | 7/7 | 1.3047±0.4823 | 2.1 |
| Compound of formula (B) + Lenvatinib Mesylate | 5 mg/kg qd / 8 mg/kg qd | 7/7 | 0.2940±0.1714*** | 77.9 |
| Compound of formula (B) + Palbociclib | 5 mg/kg qd / 50 mg/kg qd | 7/7 | 0.1439±0.0985*** | 89.2 |
| Note: compared to the solvent group, **P≤0.01, ***P≤0.001. | | | | |

**7 Conclusion**

The experimental results showed that the combined administration of a compound of formula (B) and lenvatinib mesylate or palbociclib had inhibitory effect on NU/NU mice Hep3B (human hepatocarcinoma cell line) xenograft model, which was significantly superior to that of monotherapy and showed synergistic or enhanced effect.

**Example 3: Pharmacodynamical Assay of Combined Administration of Compound of Formula (B) and Sorafenib Tosylate or Palbociclib on BALB/c Nude Mice Hep3B (Human Hepatocarcinoma Cell Line) Xenograft Model**

**1. Test Animals**

36 BALB/c Nude mice, female, 18-20 g in body weight.

**2. Test Objective**

To investigate the efficacy of compound of formula (B) in combination with sorafenib tosylate or palbociclib on Hep3B xenograft model.

**3. Drugs**

Compounds of formula (B), sorafenib tosylate, palbociclib.

Solvent: Compound of formula (B): 0.5% methylcellulose aqueous solution containing 0.4% Tween 80;
Sorafenib tosylate/palbociclib: a solution of castor oil, anhydrous ethanol and ultrapure water in a volume ratio of 1:1:6.

**4. Test Method**

4.1 Model Preparation

Same as Example 2.

4.2 Method of Administration

Monotherapy group (compound of formula (B), sorafenib tosylate, palbociclib) and combined administration

group (compound of formula (B) + sorafenib tosylate, compound of formula (B) + palbociclib), once daily by gavage; volume of administration was 10 mL/kg. In the solvent group, the solvents were administered at the same frequency and volume for 16 consecutive days.

4.3 Observation Criteria and Evaluation Criteria: Same as Example 2

**5 Statistical Method**

Same as Example 2.

**6 Results**

**6.1 Effect on Tumor Growth**

The results showed that: compared to the model (vehicle) group at corresponding time points, d13-d16, compound of formula (B) 5 mg/kg qd can significantly inhibit tumor volume growth (P<0.05); d3-d6, d10-d16, combined administration of compound of formula (B) 5 mg/kg qd + sorafenib tosylate 8 mg/kg qd significantly inhibited tumor volume growth (P<0.05); d6-d16, combined administration of compound of formula (B) 5 mg/kg qd + palbociclib 50 mg/kg qd significantly inhibited tumor volume growth (P<0.01). At the end of the test, the tumor growth inhibition rates (TGI%) of monotherapy with the compound of formula (B), monotherapy with sorafenib tosylate, monotherapy with palbociclib and combined administration with the compound of formula (B) and sorafenib tosylate or palbociclib were 30.9%, 31.4%, 44.5%, 51.0% and 87.7%, respectively, and the relative tumor volume proliferation rates (T/C%) thereof were 84.5%, 91.3%, 75.1%, 68.5% and 30.9%, respectively. Tumor observations for each group are shown in Table 3 and Figure 2 in detail.

The results showed that, at the test dose, monotherapy with sorafenib tosylate and monotherapy with palbociclib both failed to inhibit the growth of xenograft tumor (P > 0.05), while monotherapy with the compound of formula (B) (P≤0.05), and the combined administration with the compound of formula (B) and sorafenib tosylate or palbociclib (P≤0.05 or P≤0.001) can significantly inhibit the growth of the xenograft tumor respectively, wherein the effect of inhibiting tumor of combined administration of the compound of formula (B) and sorafenib tosylate or palbociclib was significantly superior to that of monotherapy. In addition, when the compound of formula (B) was administered in combination with sorafenib tosylate or palbociclib, the change rate in animal weight thereof was significantly less than that of solvent group and monotherapy group.

**Table 3** Effect of Combined Administration of Compound of Formula (B) and Sorafenib Tosylate or Palbociclib on Tumor Growth (Mean ± SD, n = 6)

| Group | Dose | Number of Animals (Survived/ Total) | Change Rate in Weight (%) | TV(mm3) d16 | T/C d16 (%) | TGI d16 (%) |
|---|---|---|---|---|---|---|
| VehicleGroup | - | 6/6 | -13.7 | 963.8±289.1 | --- | --- |
| Sorafenib Tosylate | 8 mg/kg qd | 6/6 | -17.5 | 717.2±222.7 | 84.5 | 30.9 |
| Palbociclib | 50 mg/kg qd | 6/6 | -24.7 | 711.6±313.8 | 91.3 | 31.4 |
| Compound of formula (B) | 5 mg/kg qd | 6/6 | -12.0 | 608.1±293.4* | 75.1 | 44.5 |
| Compound of formula (B) + Sorafenib Tosylate | 5 mg/kg qd / 8 mg/kg qd | 6/6 | -10.0 | 555.4±245.2* | 68.5 | 51.0 |
| Compound of formula (B) + Palbociclib | 5 mg/kg qd /50 mg/kg qd | 6/6 | -1.6 | 261.1±111.8*** | 30.9 | 87.7 |
| Note: compared to the vehicle group, *P<0.05, ***P<0.001. | | | | | | |

### 6.2 Tumor Weight

Compared with the vehicle (model) group, the tumor weights of monotherapy group of the compound of formula (B) and combined administration group of the compound of formula (B) and sorafenib tosylate or palbociclib were both significantly reduced ($P < 0.05$ or $P < 0.01$). The tumor weight inhibition rates (%) of monotherapy group of the compound of formula (B), monotherapy group of sorafenib tosylate, monotherapy group of palbociclib, and combined administration group of the compound of formula (B) and sorafenib tosylate or palbociclib were 27.8%, 19.1%, 46.4%, 57.5% and 89.2% , respectively.

The results showed that, at the test dose, monotherapy with sorafenib tosylate and monotherapy with palbociclib both failed to inhibit tumor growth ($P > 0.05$), while monotherapy with the compound of formula (B) ($P<0.05$), and the combined administration with the compound of formula (B) and sorafenib tosylate or palbociclib ($P<0.01$) can significantly inhibit tumor growth respectively, and the effect of inhibiting tumor of combined administration of the compound of formula (B) and sorafenib tosylate or palbociclib was significantly superior to that of monotherapy. See Table 4 for details.

**Table 4** Effect of Compound of formula (B) in Combination with Sorafenib Tosylate or Palbociclib on Tumor Weight (Mean ± SD, n = 6)

| Group | Dose | Number of Animals (Survived/Total) | Tumor Weight (g) | Tumor Weight Inhibition Rate (%) |
|---|---|---|---|---|
| Vehicle Group | - | 6/6 | 1.10±0.31 | --- |
| Sorafenib Tosylate | 8 mg/kg qd | 6/6 | 0.79±0.23 | 27.8 |
| Palbociclib | 50 mg/kg qd | 6/6 | 0.89±0.30 | 19.1 |
| Compound of formula (B) | 5 mg/kg qd | 6/6 | 0.59±0.34* | 46.4 |
| Compound of formula (B) + Sorafenib Tosylate | 5 mg/kg qd / 8 mg/kg qd | 6/6 | 0.47±0.26** | 57.5 |
| Compound of formula (B) + Palbociclib | 5 mg/kg qd / 50 mg/kg qd | 6/6 | 0.12±0.06** | 89.2 |
| Note: compared to the vehicle group, *P<0.05, **P<0.01. | | | | |

### 7 Conclusion

The experimental results showed that the compound of formula (B) in combination with sorafenib tosylate or palbociclib had inhibitory effect on Hep3B (human hepatocarcinoma cell line) xenograft model, which was significantly superior to that of monotherapy and showed synergistic or enhanced effect.

All publications and patents mentioned in the disclosure are incorporated herein by reference. The various modifications and variations of methods, compositions, and uses thereof described herein will be apparent to those skilled in the art without departing from the scope and spirit of the disclosure. While the disclosure has been described by specific embodiments, those skilled in the art will also understand that the disclosure should not be limited to these specific embodiments. Indeed, various variations of the described modes for implementing the disclosure that will be apparent to those skilled in the relevant art are intended to be included within the scope of the appended claims.

## Claims

1. A pharmaceutical combination comprising a compound of formula (I) or a pharmaceutically acceptable form thereof and at least one other anticancer agent, wherein the other anticancer agent is selected from a CDK inhibitor or a pharmaceutically acceptable salt thereof, lenvatinib or a pharmaceutically acceptable salt thereof, sorafenib or a pharmaceutically acceptable salt thereof, or any combination of the foregoing,

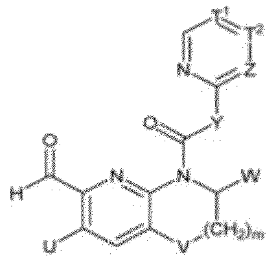

(I) ,

wherein:

$T^1$ is $CR^1$, wherein $R^1$ is cyano;
$T^2$ is $CR^2$, wherein $R^2$ is selected from the group consisting of:

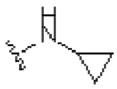 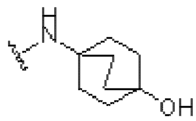 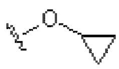 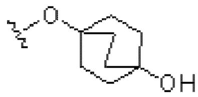 ;

Z is CH;
Y is NR, wherein R is hydrogen;
W is hydrogen;
V is $CH_2$;
U is selected from the group consisting of:

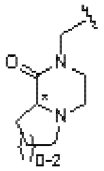 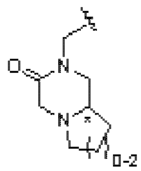 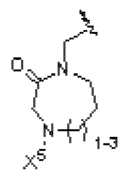 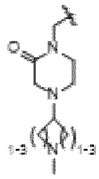 ;

wherein $X^5$ is $C_{1-4}$ alkyl;
m is 1.

**2.** The pharmaceutical combination according to claim 1, wherein $R^2$ is:

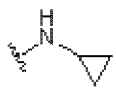

, and U is:

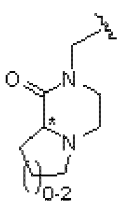

.

**3.** The pharmaceutical combination according to claim 1 or 2, wherein the compound of formula (I) is a compound of formula (A):

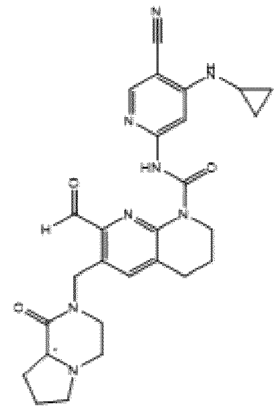

(A).

4. The pharmaceutical combination according to claim 1 or 2, wherein the compound of formula (I) is a compound of formula (B):

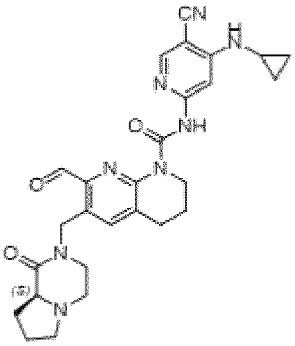

(B).

5. The pharmaceutical combination according to any one of claims 1 to 4, wherein the pharmaceutically acceptable form of the compound of formula (I) includes: a solvate, a hydrate, a pharmaceutically acceptable salt, a stereoisomer, a tautomer, an isotopic derivative, a co-crystal, a polymorph, a prodrug, and a metabolite of the compound of formula (I).

6. The pharmaceutical combination according to any one of claims 1 to 5, wherein the other anticancer agent is a CDK4/6 inhibitor or a pharmaceutically acceptable salt thereof, preferably selected from: palbociclib, ribociclib, abemaciclib, trilaciclib, G1T-38, G1T-28, AT-7519, FLX-925, and alvocidib, or a pharmaceutically acceptable salt thereof, more preferably palbociclib.

7. The pharmaceutical combination according to any one of claims 1 to 5, wherein the other anticancer agent is lenvatinib mesylate or sorafenib tosylate.

8. The pharmaceutical combination according to any one of claims 1 to 7, wherein the pharmaceutical combination comprises the compound of formula (I) or a pharmaceutically acceptable form thereof as a first dosage form, and the first dosage form of the compound of formula (I) or a pharmaceutically acceptable form thereof further comprises at least one pharmaceutically acceptable carrier, vehicle, or excipient.

9. The pharmaceutical combination according to any one of claims 1 to 8, wherein the pharmaceutical combination comprises the other anticancer agent as a second dosage form, and the second dosage form of the other anticancer agent further comprises at least one pharmaceutically acceptable carrier, vehicle, or excipient.

10. The pharmaceutical combination according to any one of claims 1 to 9 for use in treating a malignant tumor disease.

11. The pharmaceutical combination according to claim 10, wherein the compound of formula (I) or a pharmaceutically acceptable form thereof improves an efficacy of the other anticancer agent in treating a malignant tumor disease.

12. The pharmaceutical combination according to claim 10 or 11, wherein the malignant tumor is a solid tumor, preferably

selected from: lung cancer, bladder cancer, breast cancer, gastric cancer, hepatic cancer, salivary gland sarcoma, ovarian cancer, prostate cancer, cervical cancer, epithelial cell cancer and pancreatic cancer.

**13.** The pharmaceutical combination according to any one of claims 10 to 12, wherein the malignant tumor is hepatocellular carcinoma or intrahepatic cholangiocarcinoma.

**14.** The pharmaceutical combination according to any one of claims 10 to 13, wherein the malignant tumor is selected from: an advanced tumor, a refractory tumor resistant to other treatment method, a recurrent tumor after undergoing other treatment method, a metastatic tumor, a tumor mediated by FGFR4, a tumor with aberrant FGFR4 signaling pathway, and a tumor with FGR19 amplification or FGF19 overexpression.

**15.** The pharmaceutical combination according to any one of claims 1 to 14, wherein the mass ratio of the compound of formula (I) or a pharmaceutically acceptable form thereof to the other anticancer agent is 1-30:0.8-80, preferably 3-20:0.8-80, more preferably 3-12:0.8-1.2, 3-12:7.5-12.5 or 3-12:40-80, further preferably 5:8-50.

**16.** The pharmaceutical combination according to any one of claims 1 to 15, wherein a daily dose of the compound of formula (I) or a pharmaceutically acceptable form thereof is 1-1000 mg, preferably 5-500 mg, more preferably 10-300 mg, further preferably 30-200 mg, still further preferably 30-120 mg.

**17.** A pharmaceutical composition comprising a compound of formula (I) or a pharmaceutically acceptable form thereof, at least one other anticancer agent and at least one pharmaceutically acceptable carrier, vehicle, or excipient, wherein the other anticancer agent is a CDK inhibitor or a pharmaceutically acceptable salt thereof, lenvatinib or a pharmaceutically acceptable salt thereof, sorafenib or a pharmaceutically acceptable salt thereof, or any combination of the foregoing,

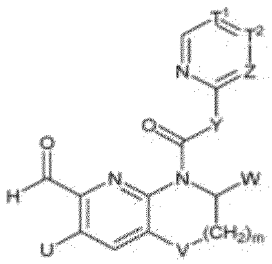
(I) ,

wherein:

$T^1$ is $CR^1$, wherein $R^1$ is cyano;
$T^2$ is $CR^2$, wherein $R^2$ is selected from the group consisting of:

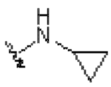
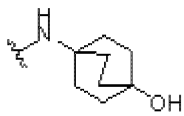
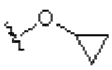
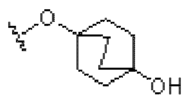
;

Z is CH;
Y is NR, wherein R is hydrogen;
W is hydrogen;
V is $CH_2$;
U is selected from the group consisting of:

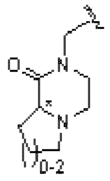 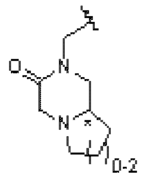 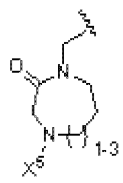 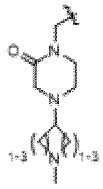 ;

wherein $X^5$ is $C_{1-4}$ alkyl;
m is 1.

**18.** The pharmaceutical composition according to claim 17, wherein $R^2$ is:

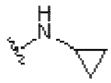

,

and U is:

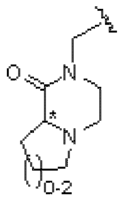

.

**19.** The pharmaceutical composition according to claim 17 or 18, wherein the compound of formula (I) is a compound of formula (A):

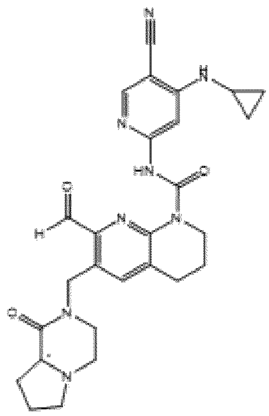

(A).

**20.** The pharmaceutical composition according to claim 17 or 18, wherein the compound of formula (I) is a compound of formula (B):

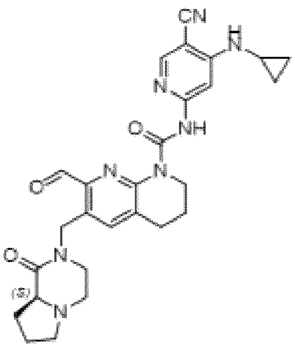

(B).

**21.** The pharmaceutical composition according to any one of claims 17 to 20, wherein the pharmaceutically acceptable form of the compound of formula (I) includes: a solvate, a hydrate, a pharmaceutically acceptable salt, a stereoisomer, a tautomer, an isotopic derivative, a co-crystal, a polymorph, a prodrug, and a metabolite of the compound of formula (I).

**22.** The pharmaceutical composition according to any one of claims 17 to 21, wherein the other anticancer agent is a CDK4/6 inhibitor or a pharmaceutically acceptable salt thereof, preferably selected from: palbociclib, ribociclib, abemaciclib, trilaciclib, G1T-38, G1T-28, AT-7519, FLX-925, and alvocidib, or a pharmaceutically acceptable salt thereof, more preferably palbociclib.

**23.** The pharmaceutical composition according to any one of claims 17 to 21, wherein the other anti-cancer agent is lenvatinib mesylate or sorafenib tosylate.

**24.** The pharmaceutical composition according to any one of claims 17 to 23 for use in treating a malignant tumor disease.

**25.** The pharmaceutical composition according to claim 24, wherein the malignant tumor is a solid tumor, preferably selected from: lung cancer, bladder cancer, breast cancer, gastric cancer, hepatic cancer, salivary gland sarcoma, ovarian cancer, prostate cancer, cervical cancer, epithelial cell cancer and pancreatic cancer.

**26.** The pharmaceutical composition according to claim 24 or 25, wherein the malignant tumor is hepatocellular carcinoma or intrahepatic cholangiocarcinoma.

**27.** The pharmaceutical composition according to any one of claims 24 to 26, wherein the malignant tumor is selected from: an advanced tumor, a refractory tumor resistant to other treatment method, a recurrent tumor after undergoing other treatment method, a metastatic tumor, a tumor mediated by FGFR4, a tumor with aberrant FGFR4 signaling pathway, or a tumor with FGR19 amplification or FGF19 overexpression.

**28.** The pharmaceutical composition according to any one of claims 17 to 27, wherein the pharmaceutical composition comprises a therapeutically effective amount of the compound of formula (I) or a pharmaceutically acceptable form thereof and the other anti-cancer agent.

**29.** The pharmaceutical composition according to any one of claims 17 to 28, wherein the mass ratio of the compound of formula (I) or a pharmaceutically acceptable form thereof to the other anticancer agent is 1-30:0.8-80, preferably 3-20:0.8-80, more preferably 3-12:0.8-1.2, 3-12:7.5-12.5 or 3-12:40-80, further preferably 5:8-50.

**30.** A method for treating a malignant tumor disease, comprising administering to a subject in need thereof a therapeutically effective amount of the pharmaceutical combination of any one of claims 1 to 16 or the pharmaceutical composition of any one of claims 17 to 29.

**31.** The method according to claim 30, further comprising determining whether the subject suffers from the malignant tumor disease or receiving information that the subject suffers from the malignant tumor disease before administering the pharmaceutical combination or the pharmaceutical composition, wherein the malignant tumor disease is **characterized by** at least one biomarker selected from: fibroblast growth factor 19 (FGF19) overexpression, FGF19 amplification and fibroblast growth factor receptor 4 (FGFR4) overexpression.

**32.** The method according to claim 31, further comprising identifying that the subject has response to the administration of the pharmaceutical composition or the pharmaceutical composition after determining whether the subject suffers from the malignant tumor disease or receiving information that the subject suffers from the malignant tumor disease.

**33.** The method according to any one of claims 30 to 32, wherein the method can be used to improve an efficacy of the other anticancer agent in treating a malignant tumor disease.

**34.** The method according to any one of claims 30 to 33, wherein the malignant tumor is a solid tumor, preferably selected from: lung cancer, bladder cancer, breast cancer, gastric cancer, hepatic cancer, salivary gland sarcoma, ovarian cancer, prostate cancer, cervical cancer, epithelial cell cancer and pancreatic cancer.

**35.** The method according to any one of claims 30 to 34, wherein the malignant tumor is hepatocellular carcinoma or intrahepatic cholangiocarcinoma.

**36.** The method according to any one of claims 30 to 35, wherein the malignant tumor is selected from: an advanced tumor, a refractory tumor resistant to other treatment method, a recurrent tumor after undergoing other treatment method, a metastatic tumor, a tumor mediated by FGFR4, a tumor with aberrant FGFR4 signaling pathway, and a tumor with FGR19 amplification or FGF19 overexpression.

**37.** A kit comprising the pharmaceutical combination of any one of claims 1 to 16, or the pharmaceutical composition of any one of claims 17 to 29, and an instruction for use.

**38.** The kit according to claim 37 for use in treating a malignant tumor disease.

**39.** The kit according to claim 38, wherein the malignant tumor is a solid tumor, preferably selected from: lung cancer, bladder cancer, breast cancer, gastric cancer, hepatic cancer, salivary gland sarcoma, ovarian cancer, prostate cancer, cervical cancer, epithelial cell cancer and pancreatic cancer.

**40.** The kit according to claim 38 or 39, wherein the malignant tumor is hepatocellular carcinoma or intrahepatic cholangiocarcinoma.

**41.** The kit according to any one of claims 38 to 40, wherein the malignant tumor is selected from: an advanced tumor, a refractory tumor resistant to other treatment method, a recurrent tumor after undergoing other treatment method, a metastatic tumor, a tumor mediated by FGFR4, a tumor with aberrant FGFR4 signaling pathway, and a tumor with FGR19 amplification or FGF19 overexpression.

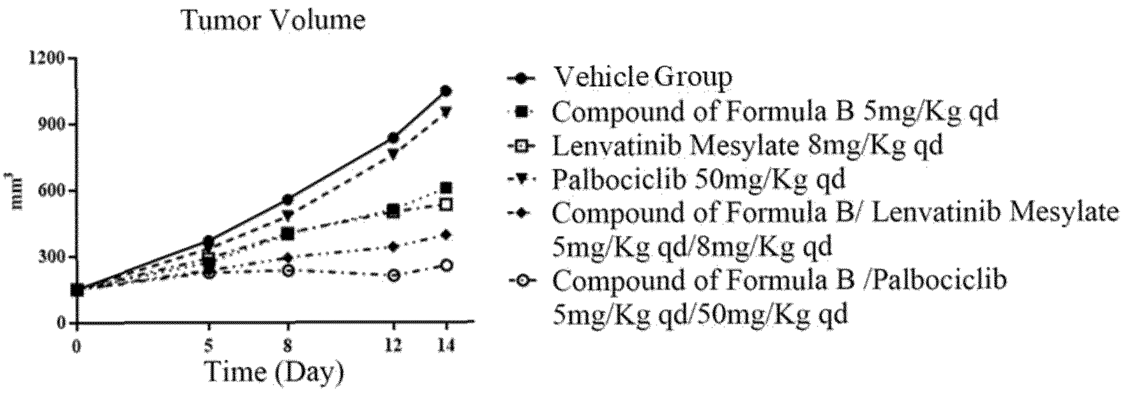
Tumor Volume
1200
900
600
300
0
mm3
0
5
8
12
14
Time (Day)
Vehicle Group
Compound of Formula B 5mg/Kg qd
Lenvatinib Mesylate 8mg/Kg qd
Palbociclib 50mg/Kg qd
Compound of Formula B/ Lenvatinib Mesylate 5mg/Kg qd/8mg/Kg qd
Compound of Formula B /Palbociclib 5mg/Kg qd/50mg/Kg qd

Fig. 1

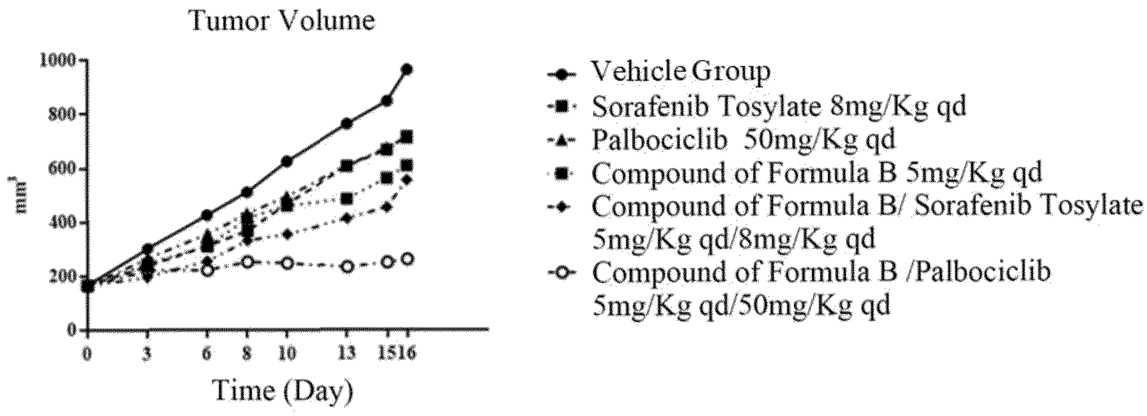
Tumor Volume
1000
800
600
400
200
0
mm3
0
3
6
8
10
13
1516
Time (Day)
Vehicle Group
Sorafenib Tosylate 8mg/Kg qd
Palbociclib 50mg/Kg qd
Compound of Formula B 5mg/Kg qd
Compound of Formula B/ Sorafenib Tosylate 5mg/Kg qd/8mg/Kg qd
Compound of Formula B /Palbociclib 5mg/Kg qd/50mg/Kg qd

Fig. 2

## INTERNATIONAL SEARCH REPORT

International application No.
**PCT/CN2023/097041**

**A. CLASSIFICATION OF SUBJECT MATTER**

A61K 31/4985(2006.01)i; A61K 31/496(2006.01)i; A61K 31/551(2006.01)i; A61K 31/517(2006.01)i; A61K 31/519(2006.01)i; A61K 31/47(2006.01)i; A61K 31/44(2006.01)i; A61P 35/00(2006.01)i

According to International Patent Classification (IPC) or to both national classification and IPC

**B. FIELDS SEARCHED**

Minimum documentation searched (classification system followed by classification symbols)

IPC:A61K,A61P

Documentation searched other than minimum documentation to the extent that such documents are included in the fields searched

Electronic data base consulted during the international search (name of data base and, where practicable, search terms used)

CNTXT, CNABS, DWPI, ENTXTC, CNKI, STN: FGFR4抑制剂, 成纤维细胞生长因子受体, 癌, 肿瘤, CDK抑制剂, 哌柏西利, 帕博西尼, 瑞博西尼, 玻玛西尼, 曲拉西利, G1T-38, G1T-28, AT-7519, FLX-925, 阿伏西地, 仑伐替尼, 乐伐替尼, 索拉非尼, FGFR, cancer, tumor, palbociclib, lenvatinib, sorafenib

**C. DOCUMENTS CONSIDERED TO BE RELEVANT**

| Category* | Citation of document, with indication, where appropriate, of the relevant passages | Relevant to claim No. |
|---|---|---|
| Y | CN 108948004 A (HANGZHOU INNOGATE PHARMA CO., LTD.) 07 December 2018 (2018-12-07)<br>see abstract, claims 1 and 8-11, and description, paragraphs 0074 and 0147-0158 | 1-41 |
| Y | US 2019192522 A1 (BLUEPRINT MEDICINES CORP.) 27 June 2019 (2019-06-27)<br>see abstract, claims 1, 3-4, 15-22 and 41, and description, paragraphs 0177 and 0229-0230 | 1-6, 8-22, 24-41 |
| Y | JI, Lin et al. "miR-486-3p Mediates Hepatocellular Carcinoma Sorafenib Resistance by Targeting FGFR4 and EGFR"<br>*Cell Death and Disease,* Vol. 11, No. (250), 20 April 2020 (2020-04-20), 1-15<br>pages 1-15, ISSN: 2041-4889 | 1-5, 7-21, 23-41 |
| Y | CN 111787922 A (EISAI R&D MANAGEMENT CO., LTD.) 16 October 2020 (2020-10-16)<br>see abstract, and claims 1-21 | 1-5, 7-21, 23-41 |
| A | WO 2021089005 A1 (CSPC ZHONGQI PHARMACEUTICAL TECHNOLOGY (SHIJIAZHUANG) CO., LTD.) 14 May 2021 (2021-05-14)<br>see abstract, and claims 1-10 | 1-41 |

☐ Further documents are listed in the continuation of Box C.   ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **28 August 2023** | **08 September 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/097041**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **30-36**
because they relate to subject matter not required to be searched by this Authority, namely:

Claims 30-36 relate to a method for treating or diagnosing a human or animal body, which falls within subject matter for which a search is not required (PCT Rule 39.1(iv)). Nevertheless, the following search is still made for claims 30-36 on the basis of the pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/097041**

| Patent document cited in search report | Publication date (day/month/year) | Patent family member(s) | Publication date (day/month/year) |
|---|---|---|---|
| CN 108948004 A | 07 December 2018 | EP 3483158 A1 | 15 May 2019 |
| | | EP 3483158 A4 | 29 July 2020 |
| | | EP 3483158 B1 | 10 August 2022 |
| | | KR 20190038485 A | 08 April 2019 |
| | | US 2020199120 A1 | 25 June 2020 |
| | | US 11352353 B2 | 07 June 2022 |
| | | KR 20210092804 A | 26 July 2021 |
| | | KR 102499780 B1 | 16 February 2023 |
| | | JP 2019518077 A | 27 June 2019 |
| | | JP 7008064 B2 | 25 January 2022 |
| | | WO 2017202390 A1 | 30 November 2017 |
| | | HK 1262855 A0 | 24 January 2020 |
| | | HK 1262922 A0 | 24 January 2020 |
| | | CN 109153678 B | 14 April 2020 |
| | | CN 111689959 A | 22 September 2020 |
| | | CN 111689959 B | 01 April 2022 |
| | | CN 108948004 B | 10 November 2020 |
| | | HK 1262922 A1 | 19 February 2021 |
| | | HK 1262855 A1 | 01 April 2021 |
| US 2019192522 A1 | 27 June 2019 | WO 2018049233 A1 | 15 March 2018 |
| | | WO 2018049233 A9 | 12 July 2018 |
| | | CN 110022900 A | 16 July 2019 |
| | | HK 40011660 A0 | 17 July 2020 |
| CN 111787922 A | 16 October 2020 | RU 2020126673 A | 10 February 2022 |
| | | RU 2020126673 A3 | 19 April 2022 |
| | | KR 20200121302 A | 23 October 2020 |
| | | EP 3737377 A1 | 18 November 2020 |
| | | MA 51570 A | 18 November 2020 |
| | | SG 11202006617 RA | 28 August 2020 |
| | | IL 275981 A | 31 August 2020 |
| | | MX 2020007375 A | 09 November 2020 |
| | | BR 112020014112 A2 | 01 December 2020 |
| | | AU 2019206480 A1 | 27 August 2020 |
| | | US 2020360371 A1 | 19 November 2020 |
| | | JP 2021512140 A | 13 May 2021 |
| | | WO 2019139977 A1 | 18 July 2019 |
| | | IN 202017034029 A | 18 September 2020 |
| | | CN 111787922 A | 16 October 2020 |
| | | RU 2787993 C2 | 16 January 2023 |
| | | MX 400379 B | 27 February 2023 |
| WO 2021089005 A1 | 14 May 2021 | CN 114641293 A | 17 June 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202210597667
- CN 108948004 A
- CN 110022900 A
- CN 109803684 A
- CN 111787922
- WO 2017202390 A1


### Non-patent literature cited in the description


- Pharmaceutical Salts and Co-crystals. RSC Publishing, 2012